# EUROPEAN PATENT APPLICATION

(11) **EP 1 640 898 A1**
(43) Date of publication of application: **29.03.2006**
(21) Application number: 04746875.6
(22) Date of filing: 25.06.2004
(51) Int. Cl.: G06Q 10/00

(54) **MEDICAL CARE SELF-CELL DELIVERY SUPPORT SYSTEM, MEDICAL CARE SELF-CELL DELIVERY SUPPORT FINANCIAL SYSTEM, AND THEIR METHODS**

(30) Priority: 27.06.2003 JP 2003185260
(71) Applicant: Renomedix Institute Inc., Ishikari-shi, Hokkai-do 0613241 (JP); Mitsui Sumitomo Insurance Co., Ltd., Tokyo 104-0033 (JP); Teikoku Hormone Mfg. Co., Ltd., Tokyo 108-8532 (JP); Hitachi, Ltd., Tokyo 100-8280 (JP)
(72) Inventor: SASAKI, Nobuo, Sapporo-shi, Hokkaido 0640802 (JP); HONMOU, Osamu, Sapporo-shi, Hokkaido 0640801 (JP); HAMADA, Hirofumi, Sapporo-shi, Hokkaido 0640959 (JP); GOTO, Shigeyuki c/o Mitsui Sumitomo Ins.Co., Ltd., Chuo-ku, Tokyo 1040033 (JP); HAYASHI, Nobuhiro c/o Mitsui Sumitomo Ins.Co.Ltd., Chuo-ku, Tokyo 1040033 (JP); TAKANO, Toru c/o Mitsui Sumitomo Ins. Co., Ltd., Chuo-ku, Tokyo 1040033 (JP); KONISHI, Kunihiro c/o Teikoku Horm. Mfg. Co. Ltd., Minato-ku, Tokyo 1088532 (JP); HASHIMOTO, Yasuhiro, Chiyoda-ku, Tokyo 1018010 (JP)
(74) Representative: Casalonga, Axel
(86) International application number: PCT/JP2004/009406
(87) International publication number: WO 2005/001732

(57) **Abstract**

Disclosed is a therapeutic autologous-cell delivery support system 100, in which a therapeutic autologous cell extracted from a member is reserved in reserve means 34, and an autologous-cell delivery request from the member which includes ID information and positional information is received. Membership information is retrieved in accordance with the received ID information, and the membership information and positional information of the member is output. When the reserve means is provided in plural number at geographically different locations, one reserve means having a shortest delivery time is selected in accordance with the positional information included in the derivery request. The delivery request can be issued using ID information storage means 11 for transmitting the ID information, in a simple operation, or automatically issued using a monitor of physical data.

## Description

### FIELD OF THE INVENTION

The present invention relates to a therapeutic autologous-cell delivery support system, and more specifically to a therapeutic autologous-cell delivery support system for supporting the delivery of a therapeutic autologous cell extracted from the patient and reserved in a depository facility, in such a manner that the therapeutic autologous-cell is delivered from the depository facility to the patient for the purpose of therapy in response to a delivery request from the patient. This application is based upon the following Japanese Patent Application. For those designated countries that permit the incorporation of publications by reference, the entire contents of the Japanese Patent Application are incorporated herein by reference, as a part of the description of this application.

### BACKGROUND OF THE INVENTION

In late years, a regeneration therapy has come under the spotlight. The regeneration therapy is intended to surgically join an organ or the like regenerated through artificial propagation to a body lesion beyond the regenerative capacity of the body, and available for therapy of lesions in a wide range of fields. The regeneration therapy essentially involves considerable works in preliminary stages, and thus an effective therapy support system is one of key factors in the regeneration therapy as well as therapeutic technologies.

As a support system concerning the regeneration therapy, there have been known a technique on a cell banking system and cell freezing/thawing method for allowing a client to make a request for accessing information about cells pre-extracted from the client and for delivering the cells to a plurality of companies to propagate/differentiate the cells therein or produce customized chemicals or cosmetics therein (see, for example, Japanese Patent Laid-Open Publication No. 2002-27983). The conventional system includes cell reserve means for reserving cells extracted from a client, and a memory storing information about the deposited cells, to allow the client to access the deposited cells and associated gene information via the Internet as needed. Based on this system, the client can instruct to deliver the cells to a cell-culturing company to propagate/differentiate the cells and to deliver the cells to a chemical/cosmetic manufacturing company to produce customized chemicals or cosmetics.

The conventional system is fundamentally intended to transport a deposited autologous (i.e. client's own) cell to a chemical/cosmetic manufacturing company or the like in response to a request from the client, to propagate/differentiate the cells according to an intended purpose. Thus, the system is required to take considerable time for propagating/differentiating the cells in the company after the transport, and is hardly applied to a regeneration therapy effective to occurrence of unexpected lesions in acute diseases or accidents. While the autologous cells in the system are used for acquisition of gene information, acquisition of test data on drug sensitivity, extraction of protein and/or other component from propagated autologous cells, transplant therapy, and reserving of totipotent ES cells (Embryonic Stem Cells), there is no publication disclosing an autologous cell capable of providing a therapeutic effect on emergency lesions by prompt in-vivo administration. In addition, while a realistic system management process for reducing a user's cost or the like is essentially established in actually implementing the above process, such a concern is not recognized in the conventional technique at all.

As above, there has not been developed any system for supporting a regeneration therapy using an autologous cell capable of providing a therapeutic effect on emergency lesions by prompt in-vivo administration, and a critical barrier in implementing such a therapeutic-autologous-cell-based therapy has not been overcome. Specifically, there are the following needs. User's ID and positional information should be transmitted through a simple operation to issue a request for delivering a therapeutic autologous cell from a delivery source or a depository facility of the cell to a delivery destination or a therapeutic facility, and an instruction on delivery should be transmitted to delivery means such as transport agencies in response to the delivery request. In this step, if there are a plurality of depository or therapeutic facilities, the system should be operable to select suitable one from them. Specifically, the system should be operable to select one depository facility allowing the time for transporting the cell to the delivery destination to be minimized. In consideration of a case where the patient's consciousness level is impaired or where the patient's ability to exercise or esthesis is lesioned, the delivery request should be automatically generated while preventing wrong activation. The user's positional information to be includes in the delivery request should be arranged to allow the user's position to be automatically acquired with accuracy. Further, it is required to initiate the therapeutic-autologous-cell-based therapy on the condition that other person has verified the identity of a user in an impaired consciousness level. It is also required to obtain a user's pre-agreement about the therapy. If the pre-agreement has not been obtained before the therapy, a given agreement shall be obtained from a participant of the user. The system should include a management process for allowing the system operation and emergency therapy to be performed without imposing an excessive cost on a user. Such a management process should be preferably set up by combining a membership organization and an insurance scheme. If a user is not indemnified by any insurance, an advance notification should be given to the user or the participant thereof to understand that no insurance will be paid, so as to afford an opportunity to prepare a necessary fund in early stages. The status of the steps in the system or the progress of the support operation for the therapy should be informed to a user or a participant thereof in real time via a network or the like. Furthermore, the system should be reliably activated in response to the delivery request.

### SUMMARY OF THE INVENTION

The present indention is directed to solve the above problems and satisfy the above needs.

Specifically, according to a first aspect of the present invention, there is provided a therapeutic autologous-cell delivery support system comprising: reserve means for reserving a plurality of therapeutic autologous cells which are extracted from the respective bodies of members and then individually propagated up to at least a population required for a therapy of regenerating an in-vivo injury region with in-vivo administration to each of the members, in a distinct manner with respect to each of the members; computer-based membership-information storage means for storing membership information including at least member's names in such a manner that the member's names are associated, respectively, with ID information uniquely corresponding to the members having the therapeutic autologous cells reserved in the reserve means; computer-based delivery-request receiving means for receiving a delivery request including the ID information of the member making a request for delivering the therapeutic autologous cell to a delivery destination for the purpose of the therapy, and positional information of the member, via a communication network; computer-based membership- information retrieval means for retrieving the membership information of the requesting member from the membership-information storage means, in accordance with the ID information included in the delivery request received by the delivery-request receiving means; and delivery-request output means for outputting information in association with the depositary means, the delivery-request output means being adapted to output the membership information retrieved by the membership-information retrieval means, and the positional information of the requesting member.

In the therapeutic autologous-cell delivery support system set forth in the first aspect of the present invention, each of the reserve means and the delivery-request output means may be provided in a plural number at geographically different locations. In this case, the therapeutic autologous-cell delivery support system further includes: computer-based delivery source determination means for determining the delivery destination from a plurality of pre-registered therapeutic facilities in accordance with the positional information of the requesting member included in the delivery request received by the delivery-request receiving means, so as to allow the requesting member to have the therapy adequately, and then determining suitable one of the plurality of reserve means as the delivery source; and computer-based delivery-request transfer means for transferring the retrieved membership information from the membership-information retrieval means in the form of including at least the member's name, and delivery destination information of the determined delivery destination, to the delivery-request output means associated with the determined reserve means as the delivery source. Further, the delivery- request output means is adapted to output the membership information and the delivery destination information transferred from delivery-request transfer means.

Further, the delivery source determination means may be adapted to determine as the delivery source the reserve means which is located at the geographically location allowing a delivery time to the determined delivery destination to be minimized.

The therapeutic autologous-cell delivery support system set forth in the first aspect of the present invention may further include ID information storage means for storing ID information of each of the members, and transmitting the ID information to the delivery-request receiving means via the communication network. In this case, the ID information storage means may be adapted to transmit the ID information to the communication network in response to a manual operation of each of the members.

The ID information storage means may be adapted to monitor at least either one of physical data selected from the group consisting of hart rate, breathing rate, electrocardiographic pattern, voice and physical movement, and to transmit the ID information to the communication network in response to the detection of abnormality in the physical data. Further, the ID information storage means may be adapted to output information about abnormality in the physical data in response to the detection thereof, and subsequently transmit the ID information to the communication network only if no confirmation signal is entered within a predetermined time-period after the output.

In the therapeutic autologous-cell delivery support system set forth in the first aspect of the present invention, the ID information storage means may be adapted to be wirelessly connected to the communication network.

The therapeutic autologous-cell delivery support system set forth in the first aspect of the present invention may further include position notification means for determination the location of the requesting member, and transmit the determined location as the positional information of the requesting member to the delivery-request receiving means via the communication network.

In this case, the position notification means may be adapted to be wirelessly connected to the communication network. Further, the position notification means may be adapted to determine the location of the requesting member according to GPS. Alternatively, the position notification means may be adapted to receive radiowaves from a ground radio communication facility so as to determine the location of the requesting member.

In the therapeutic autologous-cell delivery support system set forth in the first aspect of the present invention, the membership information stored in the membership-information storage means may include at least either one of identification physical-characteristic data selected from the group consisting of a facial portrait image, a fingerprint pattern, a vein pattern and an iris pattern.

The therapeutic autologous-cell delivery support system set forth in the first aspect of the present invention may further include computer-based agreement-confirmation means for confirming the presence of a pre-agreement of each of the members to use the therapeutic autologous cell, via the communication network, wherein the delivery-request output means is adapted to output the membership information and the positional information of the requesting member if the pre-agreement is confirmed to have been obtained from the requesting member, and to output the membership information and the positional information of the requesting member only after the agreement is obtained from the requesting member or a given participant of said requesting member if the pre-agreement is not confirmed to have been obtained form said requesting member.

The therapeutic autologous-cell delivery support system set forth in the first aspect of the present invention may further include delivery instruction means for determining one of a plurality of delivery means provided at geographically different locations to deriver the therapeutic autologous cell, in accordance with the positional information of the requesting member, and transmitting a delivery instruction to the determined delivery means.

In this case, the therapeutic autologous-cell delivery support system may further include status notification means for transmitting at least either one of information selected from the group consisting of the receipt time of the delivery request, the positional information included in the delivery request, the determined reserve means as the delivery source, the determined delivery means, the status of the delivery, the departure time from the delivery source, the estimated arrival time to the delivery destination, the presence of the therapy agreement and the presence of effective insurance guarantee, to the requesting member or a given participant of the requesting member via the communication network.

The therapeutic autologous-cell delivery support system set forth in the first aspect of the present invention may further include computer-based membership-application acceptance means for confirming the intention of an applicant to become a member, via the communication network, acquiring information including at least the name of the applicant as the membership information, via the communication network, allowing the applicant to enter a response to a questionnaire thereto via the communication network, and providing a feedback based on the questionnaire response to the applicant via the communication network.

In the therapeutic autologous-cell delivery support system set forth in the first aspect of the present invention, the delivery-request receiving means may include a dedicated link with the communication network to receive the delivery request, so as to allow the incoming of the delivery request to be judged in accordance with the fact that any data is received through the dedicated link. Alternatively, the delivery-request receiving means may include a link with the communication network to detect given data characterizing the delivery request, so as to allow the incoming of the delivery request to be judged in accordance with the detection of the given data.

In the therapeutic autologous-cell delivery support system set forth in the first aspect of the present invention, the therapeutic autologous cell may be used in at least either one of therapies selected from the group consisting of: cerebral infarction; cerebral hemorrhage; vertebral injury; cardiac infarction; cerebral apoplexy including subarachnoid hemorrhage; central and peripheral demyelinating diseases; central and peripheral degenerative diseases; cerebral tumor; disorders of higher brain function including dementia; psychiatric diseases; epilepsy; traumatic neurological diseases including head injury, cerebral contusion and spinal cord injury; inflammatory diseases; and cerebral injury-infectious diseases including Creutzfeldt-Jakob disease.

The therapeutic autologous cell may be a mesenchymal stem cell extracted from the member in an undifferentiated form. Alternatively, the therapeutic autologous cell may be prepared by extracting a mesenchymal stem cell from the member in an undifferentiated form, and then genetically modifying the extracted mesenchymal stem cell. The therapeutic autologous cell may also be prepared by differentiating a mesenchymal stem cell extracted from the member in an undifferentiated form, and inducing the differentiated cell into certain cell.

According to a second aspect of the present invention, there is provided a financial system for use with the therapeutic autologous-cell delivery support system set forth in the first aspect of the present invention. The financial system comprises computer-based cost-appropriation demand means for making a demand for appropriating a collected fund from the member to an owing cost, the cost-appropriation demand means being adapted to periodically make a demand for appropriating the collected fund to an administrative/maintenance cost to be paid on a regular basis, and making a demand for appropriating the collected fund to a temporary cost including a delivery cost arising from the delivery request.

In the financial system set forth in the second aspect of the present invention, the cost-appropriation demand means may be adapted to make a demand for appropriating the collected fund to a therapeutic cost related to the delivery request.

The cost-appropriation demand means may be adapted to make a demand for appropriating an indemnity of an insurance to a therapeutic cost related to the delivery request, wherein at least a part of the collected fund is appropriated to the insurance fee of the insurance. Further, the cost-appropriation demand means may be adapted to confirm whether effective indemnification is guaranteed by the insurance, in advance in accordance with the provisions of the insurance and the reason of the delivery request, when demanding for appropriating the insurance indemnity to the therapeutic cost related to the delivery request, and to give a notification to the requesting member or the participant thereof to understand that no indemnification is guaranteed by the insurance via the communication network.

The financial system set forth in the second aspect of the present invention may further include computer-based insurance-application acceptance means for confirming the intention of each of the members to take out the insurance, and acquiring at least a part of given information necessary for taking out the insurance, via the communication network.

The financial system set forth in the second aspect of the present invention may further include insurance-design providing means for transmitting typical insurance designs showing a payment including an insurance fee, a cost to be paid in connection with the delivery request and an indemnity in both cases of taking out the insurance and taking out no insurance, via the communication network.

According to a third aspect of the present invention, there is provided a method for supporting the delivery of therapeutic autologous cells, comprising the steps of: reserving a plurality of therapeutic autologous cells which are extracted from the respective bodies of members and then individually propagated up to at least a population required for a therapy of regenerating an in-vivo injury region with in-vivo administration to each of the members, in reserve means in a distinct manner with respect to each of the members; storing membership information including at least member's names in such a manner that the member's names are associated, respectively, with ID information uniquely corresponding to the members having the therapeutic autologous cells reserved in the reserve means, according to a computer; receiving a delivery request including the ID information of the member making a request for delivering the therapeutic autologous cell to a delivery destination for the purpose of the therapy, and positional information of the member, via a communication network according to a computer; retrieving the membership information of the requesting member from the membership-information storage means, in accordance with the ID information included in the delivery request received in the delivery-request receiving step, according to a computer; and outputting information in association with the depositary means, the information including the membership information retrieved in the membership-information retrieving step, and the positional information of the requesting member.

According to a fourth aspect of the present invention, there is provided a financing method for used with the method as defined in claim 30, comprising the step of making a demand for appropriating a collected fund from the member to an owing cost, according to a computer, wherein a demand for appropriating the collected fund to an administrative/maintenance cost to be paid on a regular basis is periodically made, and a demand for appropriating the collected fund to a temporary cost including a delivery cost arising from the delivery request.

The financing method as set forth in the fourth aspect of the present invention may include the step of making a demand for appropriating the collected fund to a therapeutic cost related to the delivery request. The financing method may further include the step of making a demand for appropriating an indemnity of an insurance to a therapeutic cost related to the delivery request, wherein at least a part of the collected fund is appropriated to the insurance fee of the insurance.

According to fifth aspect of the present invention, there is provided a therapeutic autologous-cell delivery support system comprising: reserve means reserving therapeutic autologous cells which are extracted from each body of a plurality of members and then propagated up to at least a population required for a therapy to regenerate an in-vivo injury region with in-vivo administration to each of the members, distinctively on a member-by-member basis; computer-based membership-information storage means storing a plurality of ID information uniquely corresponding to the respective members having the therapeutic autologous cells reserved in the reserve means, and each membership information of the members which includes at least each name of the members, in a mutually associated manner; computer-based delivery-request receiving means for receiving via a communication network a delivery request which includes the ID information of a specific one of the members who made a request for delivering the therapeutic autologous cell to a delivery destination for the therapeutic purpose, and location-specifying information for specifying a location of the requesting member; computer-based membership-information retrieval means for retrieving the membership information of the requesting member from the membership-information storage means, in accordance with the ID information included in the delivery request received by the delivery-request receiving means; and delivery-request output means association with the depositary means and adapted to output the membership information retrieved by the membership-information retrieval means, and the location-specifying information of the requesting member.

The therapeutic autologous-cell delivery support system set forth in the fifth aspect of the present invention may further include location determination means for determining a location of the requesting member in accordance with the location-specifying information.

In this therapeutic autologous-cell delivery support system, each of the reserve means and the delivery-request output means may be provided in a plural number and situated at geographically different locations. In this case, the therapeutic autologous-cell delivery support system may further includes: computer-based delivery source determination means for specifying one of a plurality of pre-registered facilities including a therapeutic facility, as a suitable delivery destination for the requesting member to receive the therapy, in accordance with the location of the requesting member determined by the location determination means, and then selecting one of the plurality of reserve means, as a suitable delivery source; and computer-based delivery-request transfer means for transferring the membership information with at least member's name, retrieved by the membership-information retrieval means, and delivery destination information about the specified delivery destination, to one of the plurality of delivery-request output means which is associated with the selected reserve means as the suitable delivery source, the associated delivery-request output means being operable to output the membership information and the delivery destination information transferred from the delivery-request transfer means.

The reserve means selected as the suitable delivery source by the delivery source determination means may be situated at a topographical location allowing a delivery time to the specified delivery destination to be minimized.

The therapeutic autologous-cell delivery support system set forth in the fifth aspect of the present invention may further include ID information storage means storing the ID information of the specific member. The ID information storage means may be adapted to transmit the ID information to the delivery-request receiving means via the communication network.

Further, the ID information storage means may comprise an external memory device storing the ID information thereon.

The external memory device may further store an ID information transmission program to be executed in response to connecting the ID information storage means to a computer terminal, so as to transmit the member's ID information to the delivery-request receiving means via the communication network.

The external memory device may further store a location-specifying information transmission program to be executed in response to connecting the ID information storage means to a computer terminal, so as to transmit a location of the computer terminal as the location-specifying information for specifying each location of the members, to the delivery-request receiving means via the communication network.

Further, the location-specifying information transmission program may be operable, when executed on the computer terminal, to prompt an operator of the computer terminal to enter information about a location of the operator, and then transmit the entered information as the location-specifying information to the delivery-request receiving means.

The external memory device may be configured as a USB key.

According to sixth aspect of the present invention, there is provided a therapeutic autologous-cell delivery support system comprising: reserve means reserving therapeutic autologous cells which are extracted from each body of a plurality of members and then propagated up to at least a population required for a therapy to regenerate an in-vivo injury region with in-vivo administration to each of the members, distinctively on a member-by-member basis; computer-based membership-information storage means storing a plurality of ID information uniquely corresponding to the respective members having the therapeutic autologous cells reserved in the reserve means, and each membership information of the members which includes at least each name of the members, in a mutually associated manner; computer-based continuously-operative location receiving means for continuously receiving location-specifying information for specifying each location of the members, via a communication network; facility-location storage means storing information about a plurality of facilities and each location of the facilities, in a mutually associated manner; location comparison means for comparing the location specified by the location-specifying information received from a specific one of the members with each location of the facilities stored on the facility-location storage means to determine whether there is a facility corresponding to the location of the specific member; delivery-request transmission means operable, in response to the determination of the location comparison means that there is a facility corresponding to the location of the specific member, to transmit a delivery request including the ID information and the location-specifying information of the specific member; computer-based membership-information retrieval means for retrieving the membership information of the specific member from the membership-information storage means, in accordance with the ID information transmitted from the delivery-request transmission means; and delivery-request output means association with the depositary means and adapted to output the membership information retrieved by the membership-information retrieval means, and the location-specifying information of the specific member.

In the therapeutic autologous-cell delivery support system set forth in the sixth aspect of the present invention, the delivery-request transmission means may be adapted to, before transmission of the delivery request, confirm whether the delivery request for the specific member may be transmitted.

Further, the delivery-request transmission means may be adapted to, before transmission of the delivery request, determine whether the delivery request should be transmitted, in accordance with a secondary index including at least either one of each expertise index of the facilities and a current time.

### BRIEF DESCRIPTION OF THE DRAWINGS

FIG 1 is a system block diagram showing a therapeutic autologous-cell delivery support system according to one embodiment of the present invention.
FIG 2 is an exemplary operational flowchart of the therapeutic autologous-cell delivery support system.
FIG 3 is an exemplary operational flowchart of the therapeutic autologous-cell delivery support system, continuing from FIG 2.
FIG 4 is an exemplary operational flowchart of the therapeutic autologous-cell delivery support system, continuing from FIG 3.
FIG 5 is an exemplary operational flowchart of the therapeutic autologous-cell delivery support system, continuing from FIG 4.
FIG. 6 is an image diagram of a member registration screen.
FIG 7 is an image diagram of a membership-fee payment-method selection screen.
FIG 8 is an image diagram of an insurance application screen.
FIG 9 is an image diagram of an explanatory-statement checking screen.
FIG 10 is an image diagram of a pre-agreement confirmation screen.
FIG 11 is an image diagram of a questionnaire screen.
FIG 12 is an image diagram of a daily-activity advice screen.
FIG 13 is an image diagram of a status notification screen.
FIG 14 is an image diagram of an insurance-design display screen (1).
FIG 15 is an image diagram of an insurance-design display screen (2).
FIG 16 is an MRI image showing the result of a test in which mesenchymal stem cells (1 × 10⁶ cells) were intravenously administrated to severe cerebral infarction (rat model of permanently occluded mesencephalic/cerebral veins).
FIG 17 is a graph showing the volume of cerebral infarction area in the result of the test in which mesenchymal stem cells (1 × 10⁶ cells) were intravenously administrated to severe cerebral infarction (rat model of permanently occluded mesencephalic/cerebral veins).
FIG 18 is a graph showing the change of survival rate after the occurrence of lesion in the result of the test in which mesenchymal stem cells (1 × 10⁶ cells) were intravenously administrated to severe cerebral infarction (rat model of permanently occluded mesencephalic/cerebral veins).
FIG 19 is a system block diagram showing a therapeutic autologous-cell delivery support system according to another embodiment of the present invention.
FIG 20 is a process flow diagram showing a process for reserving a therapeutic autologous cell in a depository facility in the therapeutic autologous-cell delivery support systems illustrated in FIGS. 1 and 19.
FIG. 21 is an exemplary flow chart of a continuous position notification process in the therapeutic autologous-cell delivery support system illustrated in FIG 19, which substitutes a part of the flowchart in FIG 3.

### DESCRIPTION OF THE PREFERRED EMBODIMENT

### [Mechanism of Therapeutic-Autologous-Cell-based Therapy for the Present Invention]

A therapeutic autologous-cell delivery support system according to the present invention is directed to support a therapy base on regenerative medical techniques using a therapeutic autologous cell without any surgical operation. The therapeutic autologous cell means a cell aggregation prepared by extracting an autologous cell from a member in an undifferentiated form and then propagating the extracted autologous cell up to a population required for the therapy. The mechanism of the therapeutic-autologous-cell-based therapy will be described as follows.

A stem cell in bone marrow includes a hematopoietic stem cell and a "mesenchymal stem cell (MSC)". Generally, the "stem cell" means an undifferentiated cell having both the ability of self-propagation and the ability of differentiating into a cell having a specific function. The hematopoietic stem cell is a stem cell which differentiates into a red blood cell, a white blood cell or a platelet. It is known that the mesenchymal stem cell differentiates into nerves through a neural stem cell, or into nerves through a stroma cell (with low efficiency), or into internal organs, or into a blood circulatory system, or into bone, cartilage, fat or muscle. While the mesenchymal stem cell is primarily used in the present invention, it is noted that the hematopoietic stem cell or any other suitable in-vivo stem cell may also be used. The mesenchymal stem cell can be isolated from a marrow cell extracted from bone marrow. While the marrow cell before isolation of the mesenchymal stem cell has slightly lower effectiveness, it can be used in the therapy as with the mesenchymal stem cell. A mesenchymal stem cell subjected to genetic modification or affected by a specific factor may also be used. It is believed that the mesenchymal stem cell may also be differentiated and induced in a cell, specifically a neural stem cell, nervous-system cell, red blood cell, white blood cell, platelet, cardiac muscle or precursor cells thereof, and these cells can be used in the therapy.

In an extraction process, marrow cells may be took from a subject under local anesthesia, preferably at several ml per extraction. The subject is a user of a therapeutic autologous-cell delivery support system 100, who has been registered as one of members after taking a given procedure in view of reliable management. As long as a particular manipulation such as immune suppression is not performed, a mesenchymal stem cell to be used in the therapy is limited to one extracted directly from the member or a cell derived therefrom to prevent immunological rejection. The extracted mesenchymal stem cell can be propagated and cultured up to a population required for an intended therapy. The propagated mesenchymal stem cell can be preserved or reserved for a long time through a given method such as freezing, and recovered to its active state to perform the therapy for the member as needed.

It has been verified that the mesenchymal stem cell can be used to perform a regeneration therapy having the following significant characteristics. In a regeneration therapy using the mesenchymal stem cell, even injuries in nervous system which have been practically unrecoverable through conventional techniques can be regenerated and cured only with in-vivo administration (intravenous administration) of the mesenchymal stem cell by injection or intravenous drip. In particular, its effectiveness on injuries in nervous system due to cerebral infarction, cerebral hemorrhage or vertebral injury has been verified. Among them, the effectiveness on cerebral infarction has been closely verified through various tests. This therapy is also effective to cardiac infarction; cerebral apoplexy including subarachnoid hemorrhage; central and peripheral demyelinating diseases; central and peripheral degenerative diseases; cerebral tumor; disorders of higher brain function including dementia; psychiatric diseases; epilepsy; traumatic neurological diseases including head injury, cerebral contusion and spinal cord injury; inflammatory diseases; and cerebral injury-infectious diseases including Creutzfeldt-Jakob disease. The therapy can be performed on site as well as at a given therapeutic facility. In view of the ability of curing the conventionally unrecoverable injuries and the ability of bringing out such effectiveness through a simple process such as intravenous administration, it can be understood how innovative this therapeutic method is. In addition, the therapeutic method allows patients to receive significant benefit from curing of the injuries in nervous system possibly causing serious damages in the patient's body, and thus would have significantly high social value. An medical action-mechanism of the regeneration therapy are believed such that the mesenchymal stem cell floatingly moves to and fixedly attaches onto an affected part (in-vivo injury region) and differentiates into a suitable cell to recover the original function of the affected part. Thus, it is believed that the regeneration therapy can be effectively used in any type of disorders and accidents causing injuries in nervous system. While the mesenchymal stem cell is fundamentally administrated into a vein one time, it may be administered plural times. Further, instead of intravenous administration, local administration or any other suitable route (intra-arterial administration, intracerebroventricular administration) may also be used.

### [Therapeutic Effect on Cerebral Infarction in Administration of Mesenchymal Stem Cell]

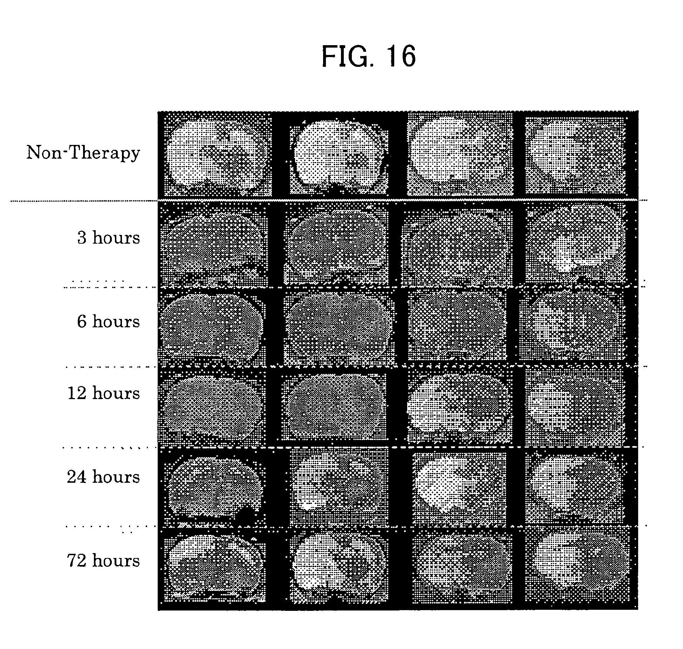

In connection with the result of an experimental test using rats, it will be described below how level an injured tissue can be recovered through the mesenchymal-stem-cell-based regeneration therapy. FIG 16 is an MRI image showing the result of a test in which mesenchymal stem cells (1 × 10⁶ cells) were intravenously administrated to severe cerebral infarction (rat model of permanently occluded mesencephalic/cerebral veins). The figure shows respective results of therapies performed under the condition that the mesenchymal stem cells were administered after each of elapsed times since the occurrence of lesion. The respective images shows the results of non-therapy, the therapy after 3 hours, the therapy after 6 hours, the therapy after 12 hours, the therapy after 24 hours and the therapy after 72 hours, in turn from upper side. Each of the images was obtained after one week since the occurrence of cerebral infarction through an MIR inspection (T₂WI). The lesion of cerebral infarction is whitely represented in the images. While the lesion of cerebral infarction is clearly observed in the non-therapy group (uppermost), only few lesions are observed in the group to which the mesenchymal stem cells were intravenously administrated after 3 hours since the occurrence of cerebral infarction. This therapeutic effect is remarkably observed as the therapy (implantation) is performed earlier. However, it is noteworthy that some level of therapeutic effect is observed even in the therapy performed after the lapse of 24 hours or more since the occurrence of cerebral infarction. The therapeutic effect is considered as a synergistic effect between a nerve protection effect and a nerve regeneration effect. The nerve protection effect or anti-edema action is enhanced as the implantation is performed earlier after the occurrence of cerebral infarction. If the therapy is performed relatively delayed, the nerve protection effect will be is relatively degraded, but the nerve regeneration effect will be reversely enhanced.

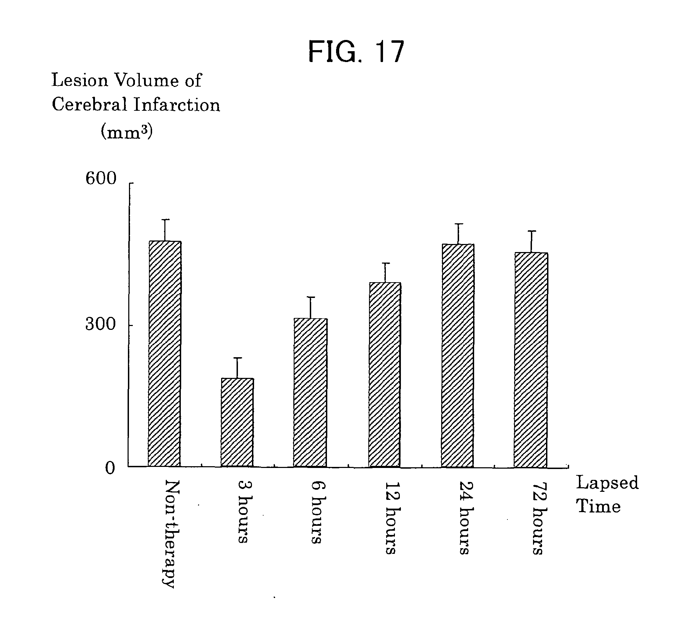

FIG 17 is a graph showing the volume of cerebral infarction area in the result of the test in which mesenchymal stem cells (1 × 10⁶ cells) were intravenously administrated to severe cerebral infarction (rat model of permanently occluded mesencephalic/cerebral veins). The figure shows respective results of therapies performed under the condition that the mesenchymal stem cells were administered after each of elapsed times since the occurrence of lesion. As seen in this graph, while the lesion is extended over an area of about 500 mm³ in the non-therapy case, it is limited to only an area of 200 mm³ in the case of therapy after 3 hours which proves a remarkable effect. As compared to the non-therapy case, the lesion volume in the cases of therapies within 12 hours is reduced as the therapy is performed earlier, and better prognosis is achieved.

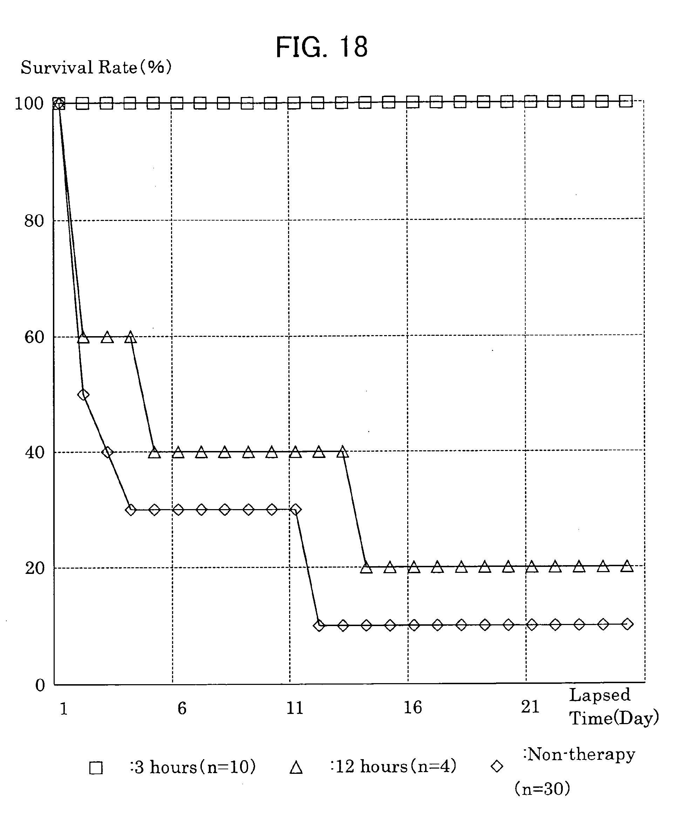

FIG 18 is a graph showing the change of survival rate after the occurrence of lesion in the result of the test in which mesenchymal stem cells (1 × 10⁶ cells) were intravenously administrated to severe cerebral infarction (rat model of permanently occluded mesencephalic/cerebral veins). The figure shows respective results of therapies performed under the condition that the mesenchymal stem cells were administered after each of elapsed times since the occurrence of lesion, wherein "n" is the number of samples. The graph proves that the therapy with intravenous administration of the mesenchymal stem cells achieves a significantly improved survive rate in severe cerebral infarction. While 90 % of the cerebral infarction model rats will die in the non-therapy case, all samples will survive if the mesenchymal stem cells are intravenously administrated within 3 hours after the occurrence of cerebral infarction. It is clearly shown that the survival rate becomes higher as the timing of initiation the therapy becomes earlier. The survival rate after therapy is also a remarkable value.

### [Outline of Support of Therapeutic-Autologous-Cell-based Therapy]

While the regeneration therapy using a mesenchymal stem cell is an innovative therapeutic method as described above, it has several restrictions. One of the restrictions is the requirement of using a stem cell pre-extracted directly from the body of a patient (or a cell derived therefrom). However, the most critical restriction is the requirement of promptly performing the therapy within a given time-period after the occurrence of lesion in a patient. A probability of obtaining an adequate therapeutic result becomes lower as the timing of initiating the therapy is delayed. On the contrary, this means that if the time between the occurrence of lesion in the patient and the initiation of the therapy is reduced, a significant effect can be obtained. Thus, in the regeneration therapy using a mesenchymal stem cell, it is a crucial point how to deliver a mesenchymal stem cell extracted directly from the body of a patient to the location of the patient within a short time after the occurrence of lesion, and this point is a major issue in implementation of this regeneration therapy. Specifically, it is required to perform the therapy within 6 hours or within 72 hours at the latest, after the occurrence of lesion (therapeutic time window). The occurrence of lesion also involves the risk of impairment in the patient's consciousness level or the patient's ability to exercise or esthesis. In order to reliably cope with such a case, a request for delivering a therapeutic autologous cell should be issued through a simple operation.

As described above, the therapeutic autologous cell acts as one kind of drug exclusive to members. However, a dedicated facility is necessary to keep the therapeutic autologous cell. Thus, it is preferable to provide a depository facility serving as a dedicated concentrated-managed facility so as to adequately keep the respective therapeutic autologous cells of a number of members. When some lesion occurs in one member, the therapeutic autologous cell should be delivered to the location of the member within a short time. Thus, such a dedicated facility is preferably provided in a plural number at geometrically different locations to allow the therapeutic autologous cell to be delivered from one of the facilities, which is nearest to the member's location. Further, since the member always requests to receive the therapy in the occurrence of an emergency lesion due to disease or accident, it is necessary to allow information of the member to be transmitted to the system through an operation as simple as possible so as to issue a delivery request. In response to the receipt of the delivery request, the system issues a delivery instruction to delivery means or adequate transport means deliver, to allow the therapeutic autologous cell to be delivered from the depository facility as a suitable delivery source to a therapeutic facility or the like as a suitable delivery destination. The therapeutic autologous-cell delivery support system 100 according to one embodiment of the present invention is operable to achieve the aforementioned objects.

The financial matter such as cost, expense or fee concerning the therapeutic autologous-cell delivery support system 100 will be described below. The operation of the therapeutic autologous-cell delivery support system 100 involves an administrative/maintenance cost to be paid on a regular basis, such as a cost for reserving the therapeutic autologous cell, and a temporary cost such as a cost for delivering the therapeutic autologous cell, and these costs should be fundamentally defrayed by the member. These costs have to be settled by collecting a fee as a membership fee from each of the member and adequately paying out of the collected fund. While the membership fee is fundamentally collected periodically, for example, monthly or biannually, a lump-sum payment such as an initial fee may be included therein in combination. If the system is arranged to collect a relatively large sum of initial fee, an operational entity can advantageously have a sufficient fund in hand, and the periodical membership fee can be relatively reduced to provide an advantage for the members. A cost concerning each of the members may be paid out of the individual collected fund of each of the members, or may be paid out of the entire collected fund of all of the members. If the system is arranged to pay out of the entire collected fund of the members, the collected fund will strongly have a meaning of inter-complement. When one of the members receives the therapeutic-autologous-cell-based therapy, it is required to pay a therapeutic cost. Thus, it is preferable to handle the therapeutic cost as one kind of the temporary cost so as to appropriate the collected fund from the member thereto. The collected fund may also be appropriated to the entire therapeutic cost of the member as well as the cost of the regeneration therapy itself. Further, the collected fund may also be appropriated to any combination of various related costs including an out-of-pocket cost in the health coverage. In the appropriation of the collected fund to the therapeutic cost or the related costs, a financial product such as an insurance having an insurance fee to which at least a part of the membership fee from each of the members is appropriated may be set up to appropriate an indemnity of the insurance to the therapeutic cost or the related costs. The insurance arranged independently of the membership-fee collection system makes it possible to clearly show how much individual pre-payment is necessary to take an advantage of the inter-complementary indemnification against an incidental cost of an intended therapy, to applicants for membership, in the form of the relationship between an indemnity and an insurance fee. The insurance fee may also be collected in a lump sum payment, in divided payments or combination thereof. As to insurance, the therapeutic autologous-cell delivery support system 100 is not necessarily required to store detailed information about paid insurance fees. Thus, the system 100 may manage only information about whether an insurance fee is adequately paid to maintain an insurance agreement effectively.

A temporary cost such as a cost for delivering the therapeutic autologous cell can become quit expensive depending on the type of delivery means 62. There is also a possibility that the membership of a certain member has not been paid for some reason. In the standpoint of the members, it is desired to allow the mesenchymal stem cell to be reliably delivered to any member who issues a delivery request, even in the above cases of having poor monetary proof. However, if the system is arranged to deliver the therapeutic autologous cell in any case, the operational entity of the therapeutic autologous-cell delivery support system 100 will have the risk of losing the fiscal soundness for maintaining the normal operation of the system. This can oppositely bring a disadvantage against the members. In order to prevent occurrence of this situation, it is contemplated to accumulate a part of the collected member fee as a pre-pooled fund, and appropriate the pre-pooled fund to a shortfall in the collected membership fee particularly when a temporary cost such as delivery cost arises. The pre-pooled fund may be managed in units of member, or in a summed fund common to the entire members, or in a combination thereof. This scheme can adequately maintain both the fiscal soundness of the operational entity of the therapeutic autologous-cell delivery support system 100 and the certainty of the therapy to the members.

### [Embodiment]

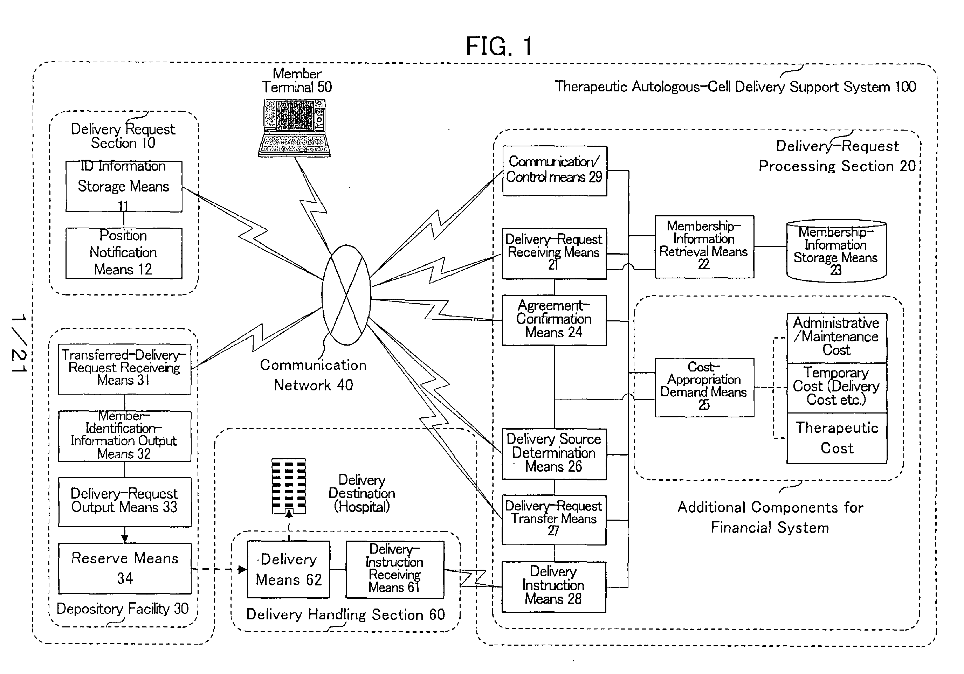

With reference to the drawings, the therapeutic autologous-cell delivery support system 100 according to one embodiment of the present invention will be described below. The construction of the therapeutic autologous-cell delivery support system 100 will be first described. FIG 1 is a system block diagram showing the therapeutic autologous-cell delivery support system 100. The therapeutic autologous-cell delivery support system 100 generally comprises a delivery request section 10, a delivery-request processing section 20, a depository facility 30, a communication network 40, and a member terminal 50. The therapeutic autologous-cell delivery support system 100 is connected with a delivery processing section 60. Preferably, the therapeutic autologous-cell delivery support system 100 is managed under a membership organization. The therapeutic autologous-cell delivery support system 100 is intended to keep a plurality of therapeutic autologous cells which are extracted from the respective bodies of members and then individually propagated up to at least a population required for a therapy of regenerating an in-vivo injury region with in-vivo administration to each of the members, and make an instruction to the delivery processing section 60 for delivering the therapeutic autologous cell in response to a delivery request issued from one of the members.

The delivery request section 10 includes ID information storage means 11 and position notification means 12. The ID information storage means 11 is a component for storing ID information, such as membership numbers, uniquely corresponding to the respective members, and outputting the ID information. The ID information storage means 11 is typically includes a small-size semiconductor chip storing information therein, and communication means for outputting the stored information. Preferably, the ID information storage means 11 has a portable configuration, such as a card shape, suitable for being carried by the member. The ID information storage means 11 is operable, responsive to a wireless read signal from outside, to output the ID information to the communication network 40. The ID information represents a request for delivering the therapeutic autologous cell, and has a function of allowing each of the members to activate the therapeutic autologous-cell delivery support system 100. The ID information storage means 11 may be configured to monitor physical data of each of the members, such as the hart rate, breathing rate, electrocardiographic pattern, voice and physical movement, and to output the ID information automatically in response to the detection of abnormality in the physical data. In this case, if one of the members looses consciousness, the physical abnormality of the member can be reliably detected to transmit the ID information. Preferably, the ID information storage means 11 is further configured such that in response to the detection of abnormality in one of the members, information about the detection of the abnormality is outputted to the member, and subsequently only if any confirmation signal (for informing that no abnormality actually occurs) is not entered from the member within a predetermined time-period after the output, the ID information is transmitted. In this case, a wrong operation caused by the erroneous detection of abnormality in the physical conditions of the member can be avoided.

The ID information storage means 11 may comprise an external memory device storing ID information, such as membership numbers. The external memory device may have any suitable configuration, such as a USB key, a SD card® or a memory stick®. When the external memory device is used, it is preferable to attach a label for emergency purposes on an outer surface thereof to indicate a cautionary statement to the effect that, when the member comes down with a disease, this external memory device should be attached/connected to a computer to initiate a process for receiving the delivery of therapeutic autologous cells. The ID information storage means 11 may also comprise a memory device incorporated in a portable telephone. More preferably, the external memory device stores a program executable when it is connected to a computer and operable to transmit the ID information of the member to the after-mentioned delivery-request receiving means through the communication network 40. Further, the computer may be configured to automatically execute this program and transmit the ID information of the member only by connecting the external memory device thereto.

The position notification means 12 is a component for outputting information about the position thereof. The position notification means 12 typically has a function of a GPS receiver, or a function of receiving a positional information service of a mobile-phone carrier, to acquire positional information and output it to the communication network 40. Preferably, the position notification means 12 has a portable configuration, such as a portable-device-like shape, suitable for being carried by each of the members. The position notification means 12 is adapted to transmit positional information of the member carrying the position notification means 12, to the communication network 40. The position notification means 12 is also adapted to transmit positional information entered by the member through member's voice or the manipulation of input bottoms or keys, to the communication network 40. The ID and the positional information of the members are basic information for the member to make a request for delivering the therapeutic autologous cell, and hereinafter referred to as "delivery request".

The position notification means 12 may comprise a mobile or portable telephone which has a function of a GPS receiver or of receiving a positional information service of a portable-phone or wireless company and a function of outputting positional information acquired therefrom. In this case, the position notification means 12 can be incorporated in a portable telephone which is widely prevalent and highly likely to be took along when leaving home, to prevent the member from forgetting to carry the position notification means 12.

The positional information of the member may be not only direct type represented, for example, by degrees of latitude and longitude, but also indirect type. The positional information may be terminal identification information for identifying a computer terminal accessed to the delivery-request processing section 20 when the position notification means 12 communicates with the delivery-request processing section 20 through a computer terminal placed at a given location. In this case, the delivery-request processing section 20 is provided with, but not shown, terminal identification means pre-storing a relationship between terminal information for specifying a computer terminal and a location where the computer terminal is placed, and identifying the location of the terminal in accordance with the terminal information obtained from the above relationship. The position notification means 12 is adapted to, when connected to a computer terminal, output terminal information for specifying the computer terminal. The position notification means 12 may comprise an external memory device storing a program operable, when the ID information storage means is connected to a computer terminal, to transmit location-specifying information for specifying a location of the member based on a location of the computer terminal. Further, considering that a computer terminal is likely to store no terminal information, the position notification means 12 may store a program for displaying a window prompting an operator of the terminal to enter information about a location of the terminal, and transmit the entered information as location-specifying information or positional information to the delivery-request receiving means. The external memory device may have any suitable configuration, such as a USB key, a SD card® or a memory stick®. When the external memory device is used, it is preferable to attach a label for emergency purposes on an outer surface thereof to indicate a cautionary statement to the effect that, when the member comes down with a disease, this external memory device should be attached/connected to a computer to initiate a process for receiving the delivery of therapeutic autologous cells. More preferably, the position notification means 12 is integrated with the ID information storage means 11 in a single unit. The delivery-request processing section 20 includes delivery-request receiving means 21, membership-information retrieval means 22, membership-information storage means 23, agreement-confirmation means 24, cost-appropriation demand means 25, delivery source determination means 26, delivery-request transfer means 27, delivery instruction means 28, and communication/control means 29. These components are not essentially constructed as individual physical configuration, but may be achieved using a computer, such as server, in such a manner that a given program is executed in the computer. The delivery-request receiving means 21 is a component for receiving a delivery request via the communication network 40. The delivery-request receiving means 21 may be typically in the form of a server, such as a Web server, connected to the communication network 40 and adapted to execute a communication application. The delivery-request receiving means 21 is adapted to automatically perform a communicate on a series of information with the delivery request section 10 which has issued a delivery request, and then automatically transmit necessary information to subsequent means in accordance with the received delivery request to reliably activate the delivery-request processing section 20. The delivery-request receiving means 21 may be configured to receive a delivery request in the form of data obtained by translating a voice signal transmitted through a public telephone network.

The membership-information retrieval means 22 is a component for managing data on membership information, and is typically in the form of a computer for managing database. The membership information includes ID information, password, name, sexuality, birth date, residence, telephone number, e-mail address, depository status of the therapeutic autologous cell (information about depository facility 30, type of the therapeutic autologous cell which is reserved therein), result of a questionnaire in an application stage, content of an insurance, the presence of pre-agreement, and a member's participant eligible to make an agreement about the therapy (in case of no pre-agreement). The membership information is used to allow a membership registration procedure to be reliably performed. The membership information may further include member-identification information representing an identification physical characteristic such as a facial portrait image, a fingerprint pattern, a vein pattern and an iris pattern. Even if one of the members looses consciousness, this information allows third party to reliably identify the member. Additionally, the membership information may include medical information of each of the members, such as blood type, result of health checkup, previous disease and status of its treatment, regularly used medicals, and drug allergy. This information can be transferred to a therapeutic facility to utilize as useful information for emergency treatment.

The membership-information storage means 23 is a component for storing the above membership information. The membership-information storage means 23 is preferably a hard disk drive storing data of the above membership information. The agreement-confirmation means 24 is a component for obtaining a pre-agreement from each of the members via a network, and obtaining an agreement during the delivery process if no pre-agreement exists. The cost-appropriation demand means 25 is a component for performing a monetary management. The cost-appropriation demand means 25 is operable to monitor that the membership fee is adequately collected from each of the members (an insurance fee is also monitored if the member takes out an insurance), and to make a demand for appropriating the contention fee to an owing cost. The cost-appropriation demand means 25 is an additional component as a part of an after-mentioned financial system capable of being used with the therapeutic autologous-cell delivery support system 100.

The delivery source determination means 26 is a component for determining a suitable delivery destination from a plurality of pre-registered therapeutic facilities in accordance with the positional information of one of members who has issued a delivery request (hereinafter referred to as "requesting member"), so as to allow the requesting member to have the therapy adequately, and then determining one of the plurality of depository facilities as a suitable delivery source.

The delivery-request transfer means 27 is a component for transferring a delivery request (membership information of the requesting member and the information about the determined delivery destination) to transferred-delivery-request receiving means 31 associated with the depository facility 30 determined by the delivery source determination means 26. The membership information to be transmitted may include the member-identification information representing the identification physical characteristic of the requesting member. The delivery request may be transmitted via the communication network 40, or may be transmitted via another network. If the depository facility 30 is provided in a single number, the delivery source determination means 26 and the delivery-request transfer means 27 may be omitted. The delivery instruction means 28 is a component for transmitting a delivery instruction for instructing to deliver the therapeutic autologous cell, to delivery-instruction receiving means 61, so as to instruct a delivery handling section 60 to handle the delivery of the therapeutic autologous cell. If the delivery means 62 is provided in a plural member, the delivery instruction means 28 is operable to select suitable one of the delivery means in accordance with the type and geometrical location of the delivery means, and transmit the delivery instruction to the determined delivery means. The communication/control means 29 is a component for performing a transmit/receive processing relative to the communication network of the delivery-request processing section 20 and associated controls. The communication/control means 29 is operable to transmit the status of the delivery to each of the member terminals 50 via the communication network 40 (or to function as status notification means), and to make a communication on information such as membership-application information, with each of the member terminals 50. Each of the members or the participant thereof can receive the information about the status of the delivery. The communication/control means 29 is connected with each of the components included in the delivery-request processing section 20, to receive their statuses and transmit them as the status of the delivery-request processing section 20 via the communication network 40.

The depository facility 30 includes transferred-delivery-request receiving means 31, member-identification-information output means 32, delivery-request output means 33, and reserve means 34. The depository facility 30 is provided in a single number or in a plural number at geographically different locations, to act as a base for reserving and delivering the therapeutic autologous cell. The transferred-delivery-request receiving means 31 is a component for receiving a delivery request transmitted from the delivery-request transfer means 27, and is typically in the form of a network client such as a Web client. The member-identification-information output means 32 is a component for outputting the member-identification information representing the identification physical characteristic in response to the receipt of the member-identification information from delivery-request transfer means 27 to inform a responsible person in the depository facility 30 of the physical characteristic for identifying the requesting member. The member-identification-information output means 32 is typically in the form of a display or speaker. The delivery-request output means 33.is operable to output the membership information of the requesting member and the delivery destination to a responsible person in the depository facility 30. The delivery-request output means 33 is typically in the form of a display or speaker, and preferably adapted to additionally output supplementary information such as map. The reserve means 34 typically has a structure including a given container for containing the respective therapeutic autologous cells of the members to refrigerate them under a low temperature (e.g. minus 196 °C which is a boiling point of liquid nitrogen at normal temperatures). The respective therapeutic autologous cells of the members are labeled to strictly reserve in a distinct manner. Further, even plural types of therapeutic autologous cells of the same member may also be reserved in a distinct manner.

The communication network 40 is a component having a network serving as a medium for transmitting information, and is typically in the form of an information communication network such as the Internet or a leased line network. If information is transmitted with voice, a public telephone network may also be used.

The member terminal 50 is a component connected with the communication network to input/output and control information, and is typically in the form of a personal computer incorporating a Web browser, a mobile or portable phone, or a portable terminal such as PDA.

Any applicant for membership, a member or a participant thereof (member's relative or the like) can make a communication on the membership information through the member terminal 50.

The delivery handling section 60 connected with the therapeutic autologous-cell delivery support system 100 includes the delivery-instruction receiving means 61 and the delivery means 62. The delivery-instruction receiving means 61 is a component for receiving a delivery instruction from the delivery instruction means 28 and outputting the delivery request, and is typically in the form of a network client such as a Web client. The delivery instruction means 28 and the delivery-instruction receiving means 61 may be connected with each other via the communication network 40. The delivery means 62 is transport means for delivering the therapeutic autologous cell reserved in the depository facility 30 to the delivery destination such as a hospital, and is typically an automobile, a two-wheeled vehicle or a helicopter. A responsible person checks the delivery instruction output from the delivery instruction means 28, and delivers the therapeutic autologous cell from the depository facility 30 to the delivery destination by use of the delivery means 62.

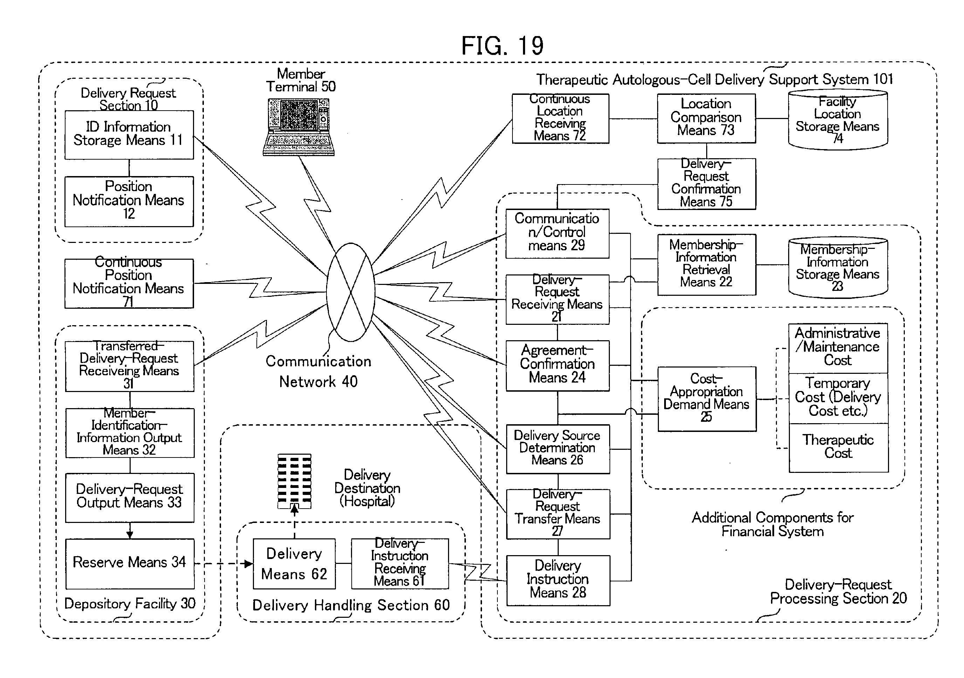

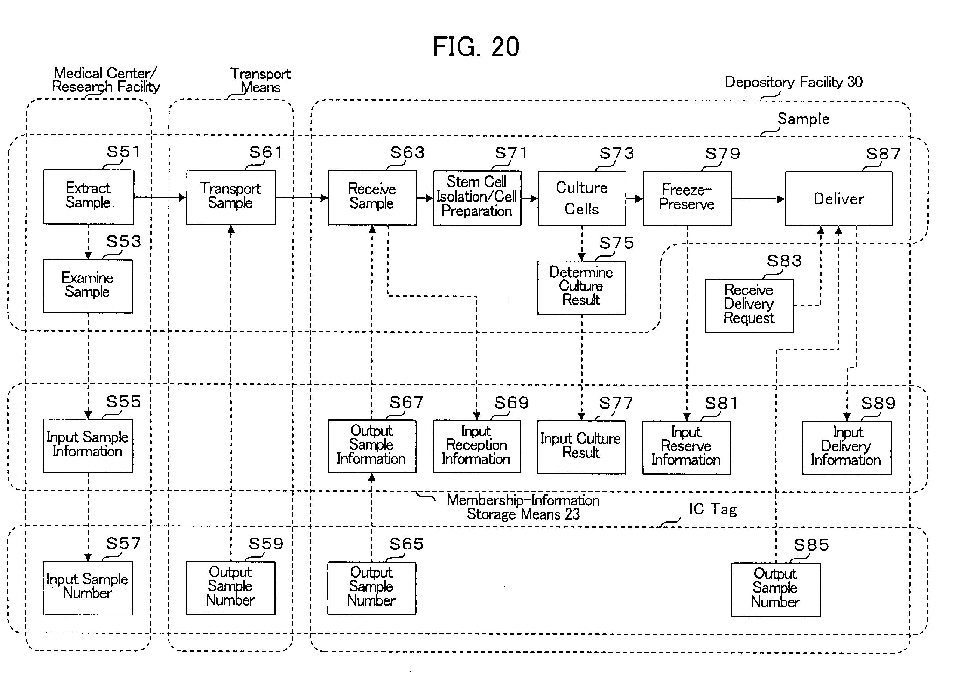

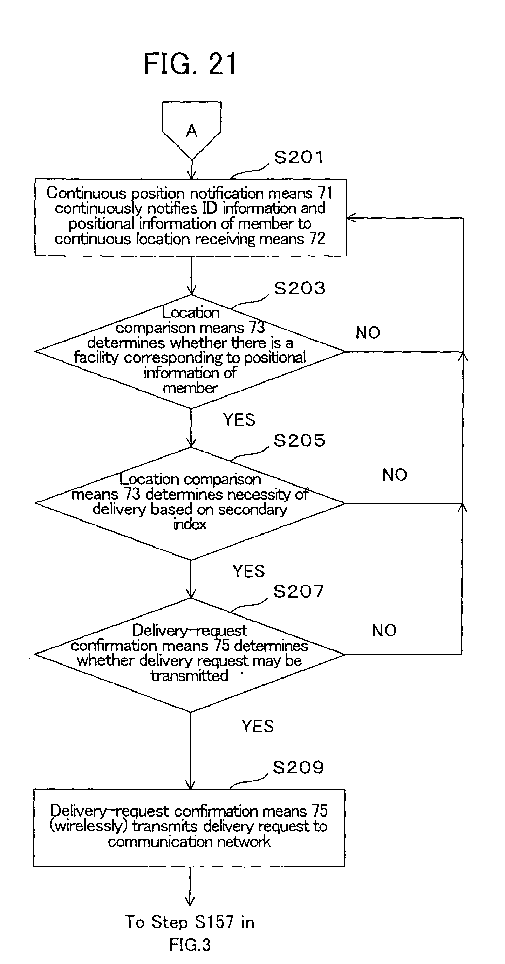

With reference to FIGS. 20 and 21, a therapeutic autologous-cell delivery support system 101 according to another embodiment of the present invention will be described below. The therapeutic autologous-cell delivery support system 101 differs from the aforementioned therapeutic autologous-cell delivery support system 100, in that the system 101 is adapted to constantly or continuously monitor positional information of a member so as to automatically determine that the member enters a facility, such as a medical center. FIG 19 is a system block diagram showing the therapeutic autologous-cell delivery support system 101. In addition to the elements or components of the therapeutic autologous-cell delivery support system 100, the therapeutic autologous-cell delivery support system 101 further comprises continuous position notification means 71, continuous location receiving means 72, location comparison means 73, facility location storage means 74 and delivery-request confirmation means 75. In FIG 19, a structurally/functionally equivalent element or component as that in the therapeutic autologous-cell delivery support system 100 is defined by the same reference numeral.

The continuous position notification means 71 is adapted to continuously output positional information thereof. Differently from the aforementioned position notification means 12 which is used for mixing positional information with a delivery request when a member transmits the delivery request, the continuous position notification means 71 is operable to notify a location of a member positively and continuously so as to allow a location of the member to be monitored. The continuous position notification means 71 typically has a function of a GPS receiver or a function of receiving a positional information service of a mobile phone company or an ad-hoc network locator, and a function of acquiring positional information therefrom and continuously outputting the acquired positional information to a communication network 40. Preferably, the continuous position notification means 71 has a shape allowing a member to carry it with him/her, such as the shape of a mobile device. Thus, positional information or location-specifying information of a member can be continuously transmitted to the communication network 40 only by carrying the continuous position notification means 71 with him/her. In order to use as the continuous position notification means 71, the position notification means 12 may be modified to be continuously operated without changing its fundamental configuration. In this case, the continuous position notification means 71 can include the position notification means 12 structurally and functionally.

The continuous location receiving means 72 is continuously connected with the continuous position notification means 71 through the communication network 40. The continuous location receiving means 72 is adapted to receive positional information or location-specifying information of a member from the continuous position notification means 71 and send the received information to the location comparison means 73.

The location comparison means 73 is adapted to compare the positional information of the member received from the continuous location receiving means 72 with each positional information of a plurality of facilities, such as medical centers, stored on facility location storage means 74, and determine whether the member is in one of the facilities each having a location stored in the facility location storage means 74. The location comparison means 73 is operable to retrieve a facility located within a given distance from the received positional information by using the received positional information as a retrieval key. In the retrieval of the positional information stored on the facility location storage means 74, each of the facilities having positional information stored on the facility location storage means 74 is regarded as a therapeutic facility capable of handling a therapy based on therapeutic autologous cells. This therapeutic facility includes emergency hospitals, general hospitals and brain surgical hospitals according to types of diseases. The location comparison means 73 is operable, when the positional information of the member is matched with the positional information of one of the facilities, to determine that the member has entered in the facility and send this information to the delivery-request confirmation means 75. In addition to the positional information, the location comparison means 73 may make the determination based on a secondary index to achieve enhanced accuracy of determination. For example, the facility location storage means 74 may store an expertise index representing degrees of expertise for each of the stored medical centers. Then, during the comparison of the position information, the location comparison means 73 may use the secondary index to make the determination on the necessity of delivery with a higher degree of accuracy. The expertise index may be set in such as manner that the highest expertise index is given to an emergency hospital specialized in therapy for cerebral infarction, and the second expertise index is given to another emergency hospital. Further, the degree of the necessity of delivery may be set at a higher value as the expertise index has a higher value. The determination may be made using these parameters. The location comparison means 73 may use a current time as one index of the determination. For example, if it is late at night or early-morning, it seems unlikely that a member goes to a medical center at such a time. Thus, the degree of the necessity of delivery may be set at a high value.

The delivery-request confirmation means 75 is operable, when the location comparison means 73 determines that a member enters in a facility, to confirm whether a delivery request for the member may be transmitted. After the confirmation, the delivery-request confirmation means 75 is operable to create a delivery request in accordance with the ID information and positional information of the member, and send the delivery request to a delivery-request processing section 20 so as to transmit the delivery request of therapeutic autologous cells. The delivery-request confirmation on transmission of the delivery request may be performed in various ways. For example, the delivery-request confirmation means 75 may automatically get on the phone or send an e-mail directly to the member and determine the necessity of the delivery request depending on a response thereto. Alternatively, the delivery-request confirmation means 75 may get on the phone or send an e-mail to a responsible person in a related facility and determine the necessity of the delivery request depending on a response thereto. Alternatively, the delivery-request confirmation means 75 may output the positional information of the member to an operator in an administrative entity of this system and obtain the confirmation of the delivery request in accordance with a delivery request determined and entered by the operator. When the confirmation on the delivery request is sought directly to a member, it may be transmitted to dedicated delivery-request confirmation receiving means which is provided adjacent to the continuous position notification means 71 and adapted to receive a delivery-request confirmation from the delivery-request confirmation means 75 and output the received delivery-request confirmation to the member. The delivery-request confirmation means 75 is operable, when the acceptance on the transmission of the delivery request is confirmed, to transmit the delivery request to the delivery-request processing section 20. The delivery request may include information about the facility determined that the member has entered therein. The information about the facility as a delivery destination allows delivery means 62 to smoothly deliver the therapeutic autologous cells. With reference to the process flow diagram in FIG 20, a process for reserving therapeutic autologous cells, or a preliminary stage of each operation of the therapeutic autologous-cell delivery support systems 100, 101, will be described below. FIG 20 is a process flow diagram showing a process for reserving therapeutic autologous cells in a depository facility 30 in the therapeutic autologous-cell delivery support systems 100, 101. In FIG 20, dotted blocks on the upper side represent a process flow about a sample (autologous cells). Dotted blocks in the median region represent a process flow about membership-information storage means 23 storing membership information, and dotted blocks on the lower side represent a process flow about an IC tag. The IC tag is attached on a container of the sample, and information for identifying the sample, such as sample number, is recorded therein. An operation for writing and reading information is performed in a non-contact manner. In FIG 20, dotted blocks on the left side represent a process flow in a medical center/research facility. Dotted blocks in the center region represent a process flow in transfer means, and dotted blocks on the right side represent a process flow in a depository facility 30.

Firstly, in a medical center/research facility, a sample (autologous cells) is taken from a member (Step S51). The sample is extracted from bone marrow in a necessary amount, for example, about 5 ml. The extracted sample is subjected to a biochemical examination to determine whether it is adequate as therapeutic autologous cells (Step S53). Biochemical information of the examined sample is stored on membership-information storage means 23 in association with ID information of the member (Step S55). A sample number for identifying the sample is entered into an IC tag attached on a container of the sample (Step S57). This sample number is stored on the membership-information storage means 23 in association with the ID information of the member. When the medical center/research facility is situated at a different location from that of the depository facility 30, the sample number of the sample is read from the IC tag (Step S59), and then the sample is transported to the depository facility 30 by transport means, such as automobile (Step S61). The transported sample is received by the depository facility 30 (Step S63). During the reception, the sample number is read from the IC tag, and checked up (Step S65). The biochemical information of the sample stored on the membership-information storage means 23 is output (Step S67), and used for a subsequent treatment for the sample. During the reception of the sample, reception information, such as receipt date, received amount and cell type, are entered into and stored on the membership-information storage means 23 (Step S69). Then, a stem cell is isolated from the sample to prepare cells (Step S71). Then, the sample is propagated by cell culture, up to at least a population required for an intended therapy (Step S73). Typically, the required population is about 10⁸ cells. The cultured autologous cells are examined to determine whether they are adequate as therapeutic cells (Step S75). The examination result of the cultured cells is entered into and stored on the membership-information storage means 23 (Step S77). The cultured autologous cells determined to be adequate as therapeutic cells are put in the reserve means together with the container, as therapeutic autologous cells, and reserved in a frozen state (Step S79). The reserve means is a refrigeration facility controlled at a given low temperature. Reserve information, such as reserve initiation date, reserve period, reserve amount and cell type, are entered into and stored on the membership-information storage means 23 (Step S81). In this state, the therapeutic autologous cells can be reserved for a long period of time.

When a delivery request from the member is received by the depository facility 30 (Step S83), the sample number is read from the IC tag to identify the therapeutic autologous cells (Step S85). The identified therapeutic autologous cells are taken out of the reserve means, and delivered to a delivery destination (Step S87). The therapeutic autologous cells in a frozen state may be unfrozen before the delivery or may be unfrozen after arrival at the delivery destination. Preferably, the therapeutic autologous cells in a frozen state is put in a dedicated portable unfreezing device just after they are taken out of the reserve means, and unfrozen under an appropriate temperature control during transportation. In this way, the reserved therapeutic autologous cells can have a usable state in a minimum time. Delivery information, such as delivery date, is entered into and stored on the membership-information storage means 23 (Step S89).

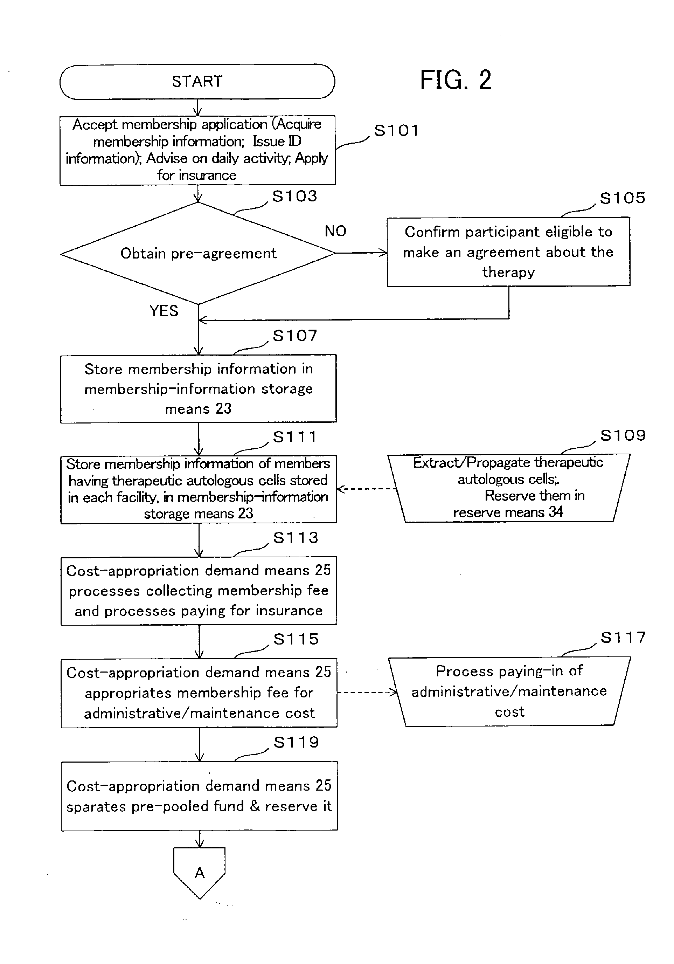

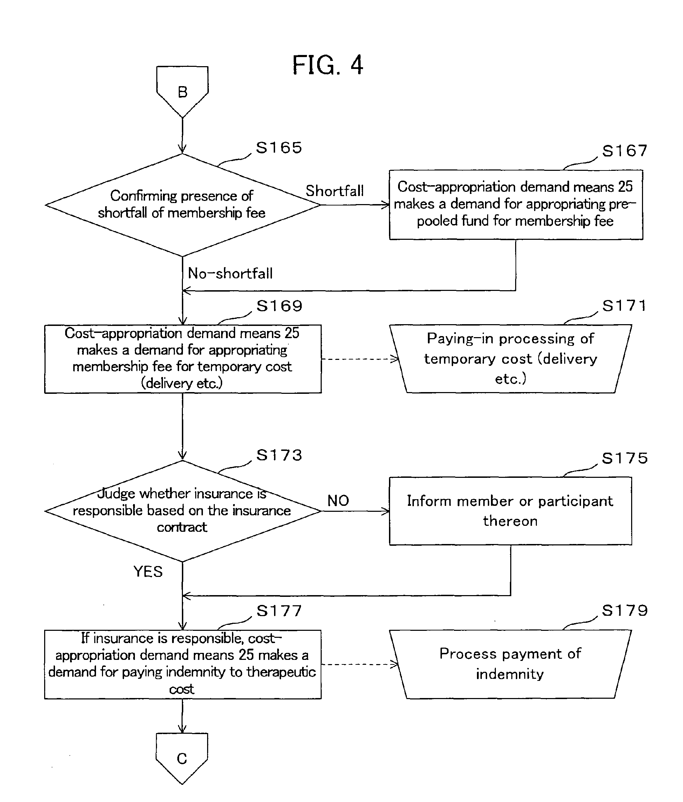

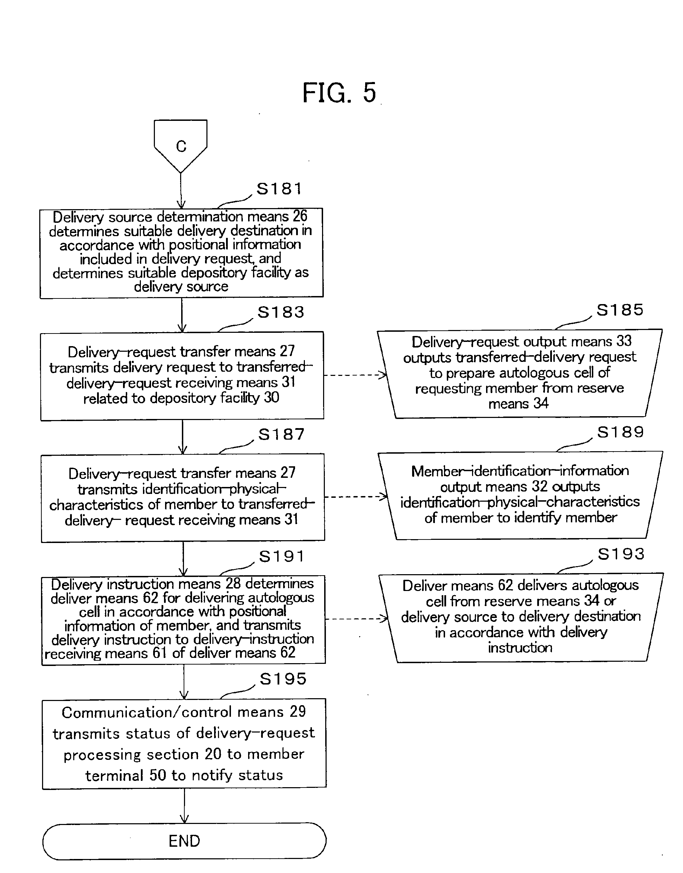

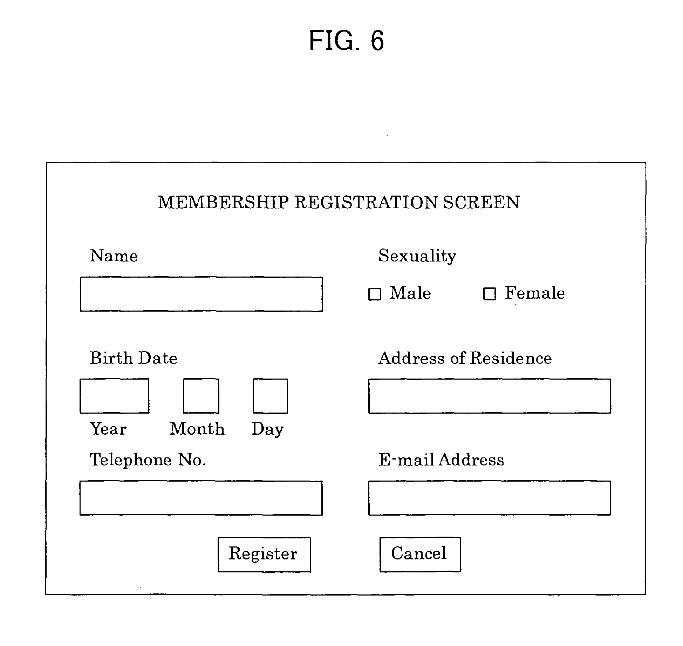

The operation of the therapeutic autologous-cell delivery support system 100 (hereinafter referred to as "support system 100" by brevity) with reference to operational flow charts. FIGS. 2 to 5 are flow charts showing a typical operation of the support system 100. FIGS. 6 to 15 are image diagrams of primary screens. The process from a membership-application acceptance to a routine operation will be first described below. The application for membership is performed on the screen of the member terminal 50 via the communication network 40, typically via the Internet.

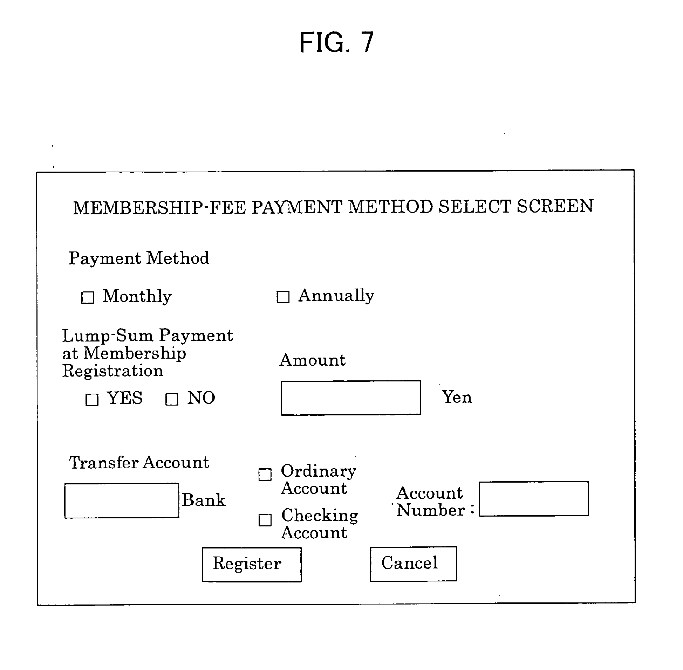

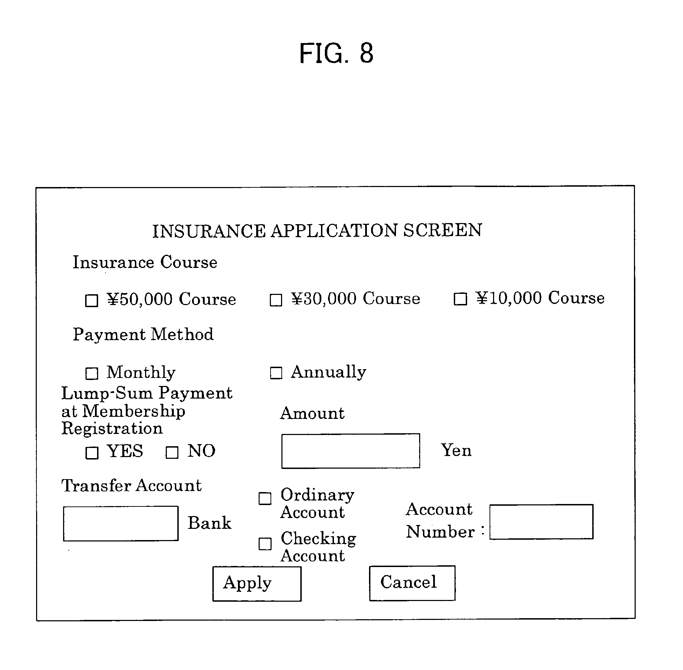

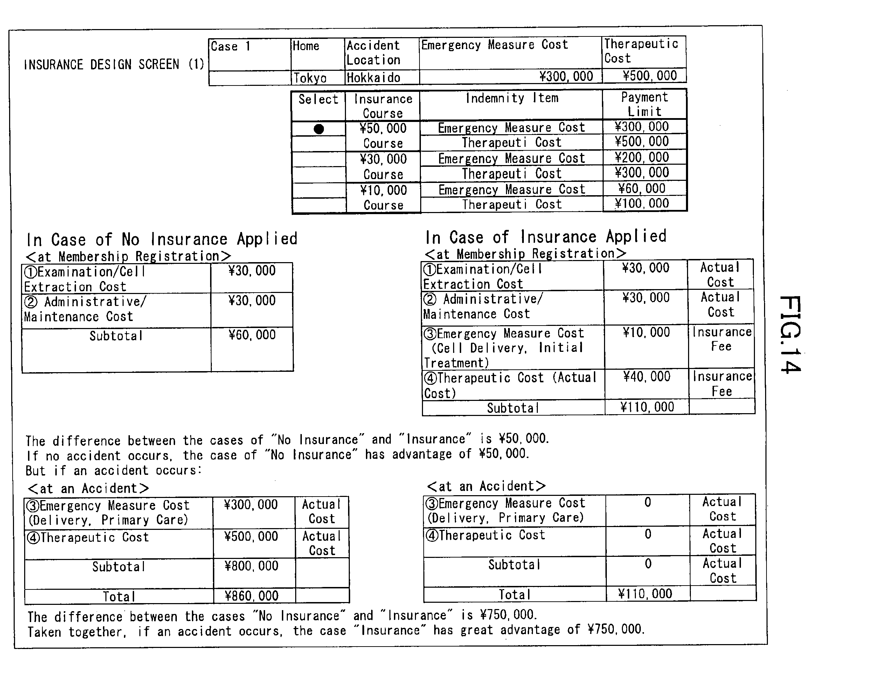

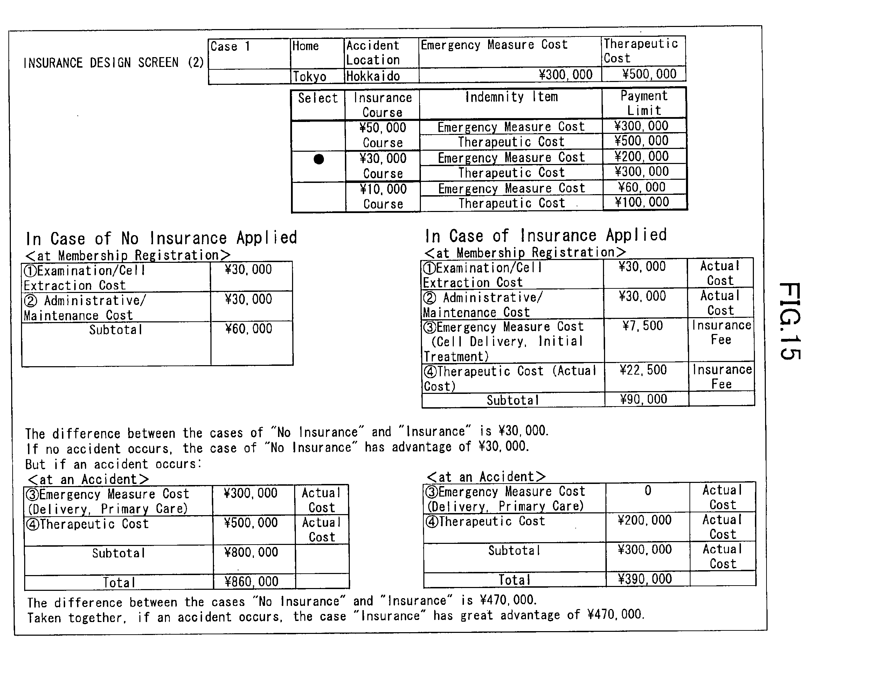

The communication/control means 29 first provides a membership-registration Website for performing a procedure of getting in the support system 100, on the communication network 40 or the Internet. When an applicant for membership accesses the support system 100 from the member terminal 50 through the membership-registration Website via the communication network 40, the communication/control means 29 is operable to display a screen for membership registration on the member terminal 50, and acquire necessary data for membership information through the membership-registration screen (Step S101). The following process is performed in the application for membership. The support system 100 issues ID information, such as a membership number, uniquely corresponding to one member, and prompts the applicant to determine a password. Then, the support system 100 prompts the applicant to enter necessary items such as name, sexuality, birth date, residence, telephone number or e-mail address. FIG 6 is an image diagram of the membership-registration screen. Then, the support system 100 displays a membership-fee payment method select screen for entering payment method of membership fee (monthly, annually), selection of lump-sum payment at the membership registration and its amount, bank name, account type and account number on transfer account, and others, and prompts the applicant to enter information about such items. FIG 7 is an image diagram of the membership-fee payment method select screen. In addition to the input items on transfer account, any other input item on payment means, such as credit card, may be displayed. Further, in the application for membership, an insurance application screen for taking out an insurance for paying a temporary cost such as a therapeutic cost, a delivery cost and others by an indemnity of the insurance is also displayed. That is, the confirmation of the intension to take out the insurance is performed via the communication network 40, and at least a part of given information necessary for taking out the insurance is acquired via the communication network 40. FIG 8 is an image diagram of the insurance application screen. The insurance application screen is configured to allow the applicant to select one of a plurality of types depending on the level of indemnification. For example, the applicant can select one of three types of insurance fee: ¥ 50,000, ¥ 30,000 and ¥ 10,000. The applicant can also enter payment method (monthly, annually), selection of lump-sum payment at application and its amount, bank name, account type and account number on transfer account (or credit card number), and others. Given that three type of insurances: first one includes conditions of insurance payment limit: ¥500,000 for a therapeutic cost and ¥ 300,000 for a temporary cost such as a delivery cost, and insurance fee: ¥50,000; second one includes conditions of insurance payment limit: ¥300,000 for a therapeutic cost and ¥ 200,000 for a temporary cost, and insurance fee: ¥30,000; third one includes conditions of insurance payment limit: ¥100,000 for a therapeutic cost and ¥ 600,000 for a temporary cost, and insurance fee: ¥10,000. In order to promote the application of the insurance, it is preferable to display typical designs of insurance fee, cost arising from a delivery request (emergency measure cost at accident or therapeutic cost), and insurance payment limit or indemnity, in each case of application and non-application of the insurance so as to clarify the benefit of the application. FIG 14 is an image diagram of an insurance-design screen (1), and FIG 15 is an image diagram of an insurance-design screen (2). While a delivery cost in this example is included in the emergency measure cost at accident, the delivery cost is preferably determined by a given calculation in accordance with the residence of the applicant, the location of the nearest depository facility 30, the type of delivery means 62.

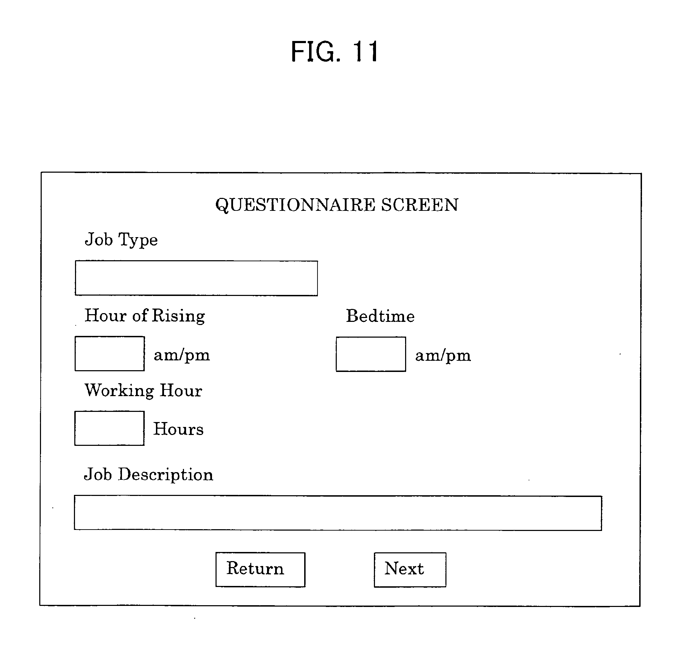

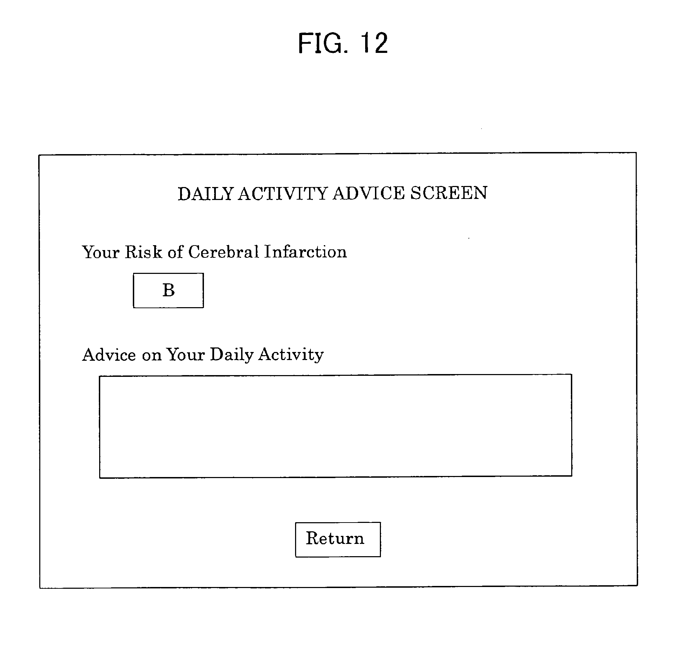

In the process of the membership registration, it is preferable to issue a given questionnaire in combination with doctor's questions. FIG 11 is an image diagram of a questionnaire screen in the above case. The questionnaire includes an item of risk factors concerning disease/accident to which the therapeutic-autologous-cell-based therapy is applicable. For example, the item includes job type, rising hour, bedtime hour, business hours, job description, amount of drinking, smoker/nonsmoker, detail of diet meal, hours of exercise, stress factor, previous cerebral infarction, previous cerebral embolism, family history of cerebral embolism, age, sexuality, previous diabetes, presence of high-blood, pressure, presence of hyperlipemia, addiction of cigarettes, addiction of alcohol, and degree of obesity. A risk contribution ratio is given to each of the questionnaire items, and the risk degree of cerebral infarction can be determined by calculation based on the entered result of the questionnaire and the risk contribution ratio. The insurance fee may be, or may not be, differentially changed depending on the questionnaire result (or the risk degree of cerebral infarction). The risk degree of cerebral infarction and the advice of daily activity may also be displayed according to the questionnaire result to carry out a simple counseling so as to correct the daily activity of the member and reduce the risk degree. In this way, it is preferable to feedback the risk degree of cerebral infarction, the advice of daily activity and others. FIG 12 is an image diagram of a daily-activity advice screen in the above case. In this figure, the risk degree of cerebral infarction is evaluated as "B". When the risk degree of cerebral infarction is high, a guide screen for an inspection, such as medical checkup of the brain, and an associated application screen may also be displayed. If the risk degree can be lowered by the effect of the daily activity advice, the payment from the collected fund to a temporary cost such as a delivery cost will be reduced to allow the collected fund or the insurance fee to be easily managed or arranged.

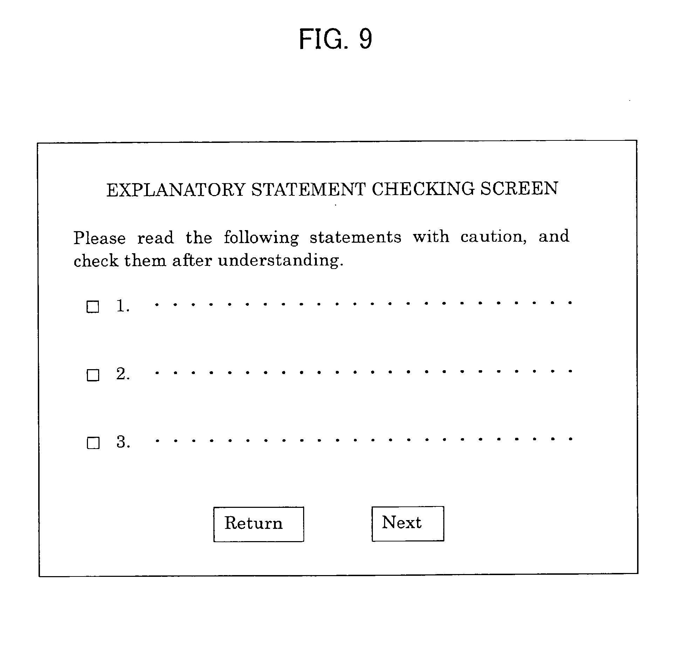

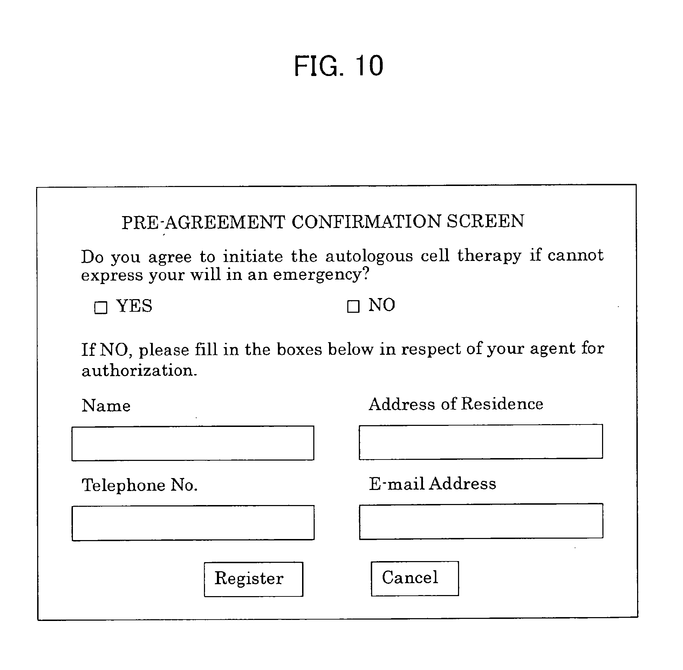

Then, the agreement-confirmation means 24 is operable to display an explanatory statement on the therapeutic-autologous-cell-based therapy, and obtain an agreement about the therapy as a part of the membership information (103). This agreement is an informed consent including a positive intention of the member to permit the therapy to be initiated regardless of the state of the member if a lesion (disease or accident) occurs. In view of the possibility that the member looses consciousness at occurrence of lesions, it is critically important to confirm the intention to the therapy in this way and obtain an agreement in advance. Preferably, the display of the explanatory statement includes a check bottom for each item of the statement, an input box for allowing a keyword in the statement to be entered therethrough, and/or a function of arranging the order of paragraphs of the statement to reproduce the statement, and prompts the member to check the bottom or enter the keyword, so as to help sufficient understanding of the applicant to the statement. FIG 9 is an image diagram of an explanatory-statement checking screen with checking buttons. If no agreement to the therapy can be obtained from the member in this stage, the agreement-confirmation means 24 will prompt the member to enter information (name, residence, telephone number (emergency contact information), e-mail address, relationship, etc) about a member's participant eligible to make an agreement about the therapy as an agent for authorization, from the member terminal 50 (Step S 105). The term "member's participant" herein means a person having a certain responsibility for the member's health, such as a member's family, a person of a company for which the member works, or a member's doctor. This makes it possible to pre-confirm that when the intention of the member cannot be confirmed at occurrence of lesions, the therapy may be initiated if an agreement is obtained from the participant (as an agent for the member), so that the support system 100 can make contact with the participant at occurrence of lesions to make an agreement about the therapy. FIG 10 is an image diagram of a pre-agreement confirmation screen for confirming the presence of a pre-agreement and entering the agent for authorization.

The membership information obtained in the above steps is stored in the membership-information storage means 23 by the membership-information retrieval means 22 (Step S 107). A therapeutic autologous cell is extracted from a new member at a given facility such as a hospital. After propagated and cultured up to a population required for the therapy, the therapeutic autologous cell is reserved in the reserve means 34 (Step 109). As the therapeutic autologous cell, a mesenchymal stem cell (MSC) is extracted from bone marrow. More specifically, the mesenchymal stem cell is isolated from an extracted marrow cell. The isolated mesenchymal stem cell is propagated up to a population required for the therapy, typically up to a cell number in the range of about 1 × 10⁷ to 1 × 10⁸. The propagated mesenchymal stem cells are controlled such that they are suspended in several dozen cc of solution. This mixture as-is may be administrated into the body of a patient. Then, the mixture is cooled down to a target temperature by a program freezer, and reserved in a container filled with liquid nitrogen. The mesenchymal stem cell may be subjected to genetic modification or be affected by a specific factor. The mesenchymal stem cell may also be differentiated and induced into a cell, specifically a neural stem cell, nervous-system cell, red blood cell, white blood cell, platelet, cardiac muscle or precursor cells thereof, and these cells can be usable as the therapeutic autologous-cell. Fundamentally, it is believe that the mesenchymal stem cell will be able to be induced into any kind of cells through the above process. Thus, plural kinds of therapeutic autologous cells may be prepared. In this case, the reserve means 34 stores the plural kinds of therapeutic autologous cells in distinct manner. If each of the depository facility 30 and the reserve means 34 is provided in a plural number at geographically different locations, one of the reserve means 34 which has the highest accessibility to the location of a requesting member will be preferably selected in consideration of the residence or any other suitable information of the requesting member. The "reserve means 34 having the highest accessibility" means that it is located at a minimum distance to a subject, or is located at a geographical location connected to the subject in the shortest time or in a minimum delivery time in view of mode of transportations and access routes.

It is understood that all or more than two of depository facilities 30 may be used. In case of business trips, a depository facility 30 which has not been used may also be temporarily used. If the location of the member is changed de to job relocation or moving house, the reserved therapeutic autologous cell may be transferred to another depository facility 30 closer to a new location. Preferably, the support system 100 is configured such that when the member accesses the support system 100 via the Internet to update the registered residence, options of new depository facilities 30 are displayed and selected, so as to allow the above transfer to be triggered by the selection.

After the completion of the reserve operation, the membership-information retrieval means 22 is operable to store information of which depository facility 30 reserves the therapeutic autologous cell of specific one of the members, in the membership-information storage means 23 (Step S111). Then, the cost-appropriation demand means 25 performs a processing of collecting the membership fee on an administrative/maintenance cost and a processing of paying an insurance fee (Step S 113). That is, the cost-appropriation demand means 25 receives related information through the communication/control means 29 to confirm that the membership fee is normally collected, and the insurance fee is normally paid. The cost-appropriation demand means 25 periodically makes a demand for appropriating the collected fund to the administrative/maintenance cost, through the communication/control means 29 (Step 115). Specifically, the cost-appropriation demand means 25 makes a demand for paying from the account for the collected membership found to an account for administrative/maintenance costs of other than the operational entity of the support system 100 by direct deposit or account transfer, for example, monthly. The operational entity having the above account for an administrative/maintenance cost performs a processing corresponding to the completion of paying-in after receiving the sent administrative/maintenance cost by direct deposit or account transfer. (Step S 117). The cost-appropriation demand means 25 performs a processing of accumulating a part of the value derived by subtracting an administrative/maintenance cost from the collected fund, as a pre-pooled fund (Step 119). Specifically, the cost-appropriation demand means 25 makes a demand for paying from the account for the collected membership found to an account for the pre-pooled fund by direct deposit or account transfer, for example, monthly.

The operation at occurrence of lesions in one of the members, the delivery request section 10 includes a monitor of physical data of the member, such as hart rate, breathing rate, electrocardiographic pattern, voice and physical movement. When a given abnormality is detected in the physical data, the monitor judges it as the occurrence of a lesion, and activates the delivery request section 10 (Step 151). Preferably, the monitor is arranged to detect an abnormality peculiar to a given disease, such as cerebral infarction, covered by the present invention. In order to prevent occurrence of a wrong operation, the delivery request section 10 may be configured such that in response to the detection of abnormality in data on the physical conditions, information about the detection of the abnormality is outputted, and subsequently only if no confirmation signal is entered within a predetermined time-period after the output, a delivery request is transmitted. Each of the members can also manually activate the delivery request section 10. While the cerebral infarction involves various symptoms from mild cases to severe cases, depending on the position of the brain injured thereby and the level thereof, major symptoms includes motor paralysis, disturbance of sensation, consciousness disorder, aphasia, dementia, involuntary movement and cardiac arrest (death). In the cerebral infarction, a patient generally remains consciousness even after the lesion is caused. Thus, even if the delivery request section 10 is designed such that it is manually activated by each of the members, any member with the region would be able to reliably activate the delivery request section 10 in most cases. The position notification means 12 included in the delivery request section 10 acquires the positional information of the requiting member (Step S 153). The positional information is obtained through the manual operation of the requesting member, the receipt of GPS signals, or the receipt of signals from a ground radio station such as a positional information service of a mobile-phone carrier. The ID information storage means 11 reads the ID information stored therein, and outputs the read ID information to the delivery-request processing section 20 via the communication network 40 (Step S155). Preferably, the read ID information is wirelessly output as a radio signal. In this process, the positional information is also transmitted. Thus, a delivery request is transmitted from a position close to the requesting member.

The delivery-request receiving means 21 receives the transmitted ID information and the positional information of the requesting member (Step S 157). In response to this receipt, the delivery-request processing section 20 is activated. The transmitted ID information and the positional information of the requesting member may be transmitted through a single data-communication operation, or may be transmitted through a plurality of data-communication operations. In this process, the delivery-request receiving means 21 itself must be autonomously activated in response to the received data, so that the delivery-request receiving means 21 reliably activates the delivery-request processing section 20 in response to the delivery request from the delivery request section 10. Thus, the delivery-request receiving means 21 is required to continuously monitor received data, and discriminate a delivery request from them. As a technique satisfying this requirement, the delivery-request receiving means 21 may be configured to receive signals only through a link dedicated to a delivery request ("link" means a link portion specified by an address of URL in WWW; a link portion specified by an e-mail address in e-mail; a link portion specified by a telephone number in telephone lines, etc), and to judge all data transmitted to its address as a delivery request. In this case, when the delivery-request receiving means 21 receives data, it determines the presence of the delivery request section 10 being issuing a delivery request in accordance with only the fact of the receipt of the data, and refers to transmitter's information in the received data to transmit a transmission-enabling signal for allowing the delivery request section 10 as the transmitter to successively transmit the ID information and positional information of the requesting member. If the communication is performed through WWW, the transmitter's information in the received data will be included in a part of header region in HTTP session for data transmission, or the like. If the communication is performed through e-mail, the transmitter's information will be included in the transmitter's e-mail address. If the communication is performed through telephone lines, the transmitter's information can be obtained using, for example, a number display service (Referring to Japanese Patent-Laid Open Publication No. 20003-18634 as a related technology).

In response to the received transmission-enabling signal, the delivery request section 10 transmits the ID information and positional information of the requesting member to the delivery-request receiving means 21. Thus, the delivery-request receiving means 21 can reliably receive the delivery request including the ID information and positional information of the requesting member, and instruct the computer to perform the following processing so as to activate a series of operations of the delivery-request processing section 20. While the above operational example has been configured such that the entire delivery request is transferred by transmitting data two or more times, the entire information of a delivery request may be included in a single transmission data to transfer the entire delivery request through a single communication operation.

The delivery-request receiving means 21 may also be configured to receive data through a link which is a specific link, but is not dedicated to a delivery request. In this case, a given data (header information etc.) characterizing a delivery request may be detected from data transmitted to the address of the specific link, to determine the arrival of a delivery request.

Based on the received ID information, the membership-information retrieval means 22 retrieves information of the requesting member from the membership-information storage means 23 (Step S159). Specifically, information about the therapeutic autologous cell of the requesting member is read using the ID information as a retrieval key. Then, agreement-confirmation means 24 confirms whether any pre-agreement about the therapy to the requesting member at Step 103 (Step 161). If the pre-agreement has been obtained, the process advances to Step 165. If no pre-agreement is obtained, an agreement about the therapy to the requesting member is obtained directly from the requesting member (when the requesting member remains consciousness) or from a participant of the requesting member (when the requesting member looses consciousness), through the member terminal via the communication network 40. Then, the process advances to Step S165.

Then, the cost-appropriation demand means 25 retrieves information about the requesting member from the membership-information retrieval means 22 and the membership-information storage means 23 to confirm whether any shortfall exists in the membership fee or whether the membership fee is adequately paid (Step S165). If some shortfall exists in the membership fee, the cost-appropriation demand means 25 will make a demand for appropriate the pre-pooled fund to the shortfall. Specifically, the cost-appropriation demand means 25 will make a demand for paying from the account for the pre-pooled fund to the account for the collected fund from the membership fee, by direct deposit or account transfer (Step S167). Thus, the risk of the deletion of membership due to the shortfall in the membership fee can be avoided, and the requesting member can reliably receive the therapy at occurrence of lesions even if the membership is not adequately paid. Subsequently, the process advances to Step S169.

If no shortfall in the membership fee is confirmed at Step S 165, the process advances directly to Step S169. Then, the cost-appropriation demand means 25 makes a demand for appropriating the collected fund to a temporary cost such as delivery cost (Step S 169). Specifically, the cost-appropriation demand means 25 makes a demand for paying from the account for collected fund to the account for the temporary cost such as the delivery cost from the membership fee, by direct deposit or account transfer. The operational entity having the account for the temporary cost such as the delivery cost performs a processing corresponding to the completion of paying-in after receiving the temporary cost such as the delivery cost by direct deposit or account transfer (Step S171). The cost-appropriation demand means 25 judges whether the insurance is applied to the therapy concerning the delivery request or whether the insurance is responsible thereto (Step S173). If the cost-appropriation demand means 25 judges that the insurance is not responsible, and no indemnity will be obtained, the communication/control means 29 will transmits this information to the member terminal 50 via the communication network 40 so as to inform the requesting member or the participant thereof of the information (Step S 175). If no indemnity is obtained, a large amount of temporary cost can be imposed on the requesting member. This process is intended to inform this risk in an early stage and promote awareness in advance of the therapy. Further, this gives the requesting member or the participant thereof a chance to show his/her intention to pre-pay the therapeutic cost which is not covered by the indemnity, so as to reduce the risk that a hospital cannot confirm the intention to pay the therapeutic cost and consequently the requesting member cannot receive the therapeutic-autologous-cell-based therapy in occurrence of lesions. Then, the process advances to Step S177. If it is judged that the insurance is responsible, at Step S 173, the process will advance directly to Step S 177. Then, the cost-appropriation demand means 25 makes a demand for paying the indemnity to be appropriated to an intervention cost (Step S177). In this step, before the demand of the payment, a step of obtaining an agreement from the contractant-side may be provided. Specifically, the cost-appropriation demand means 25 transmits information for specifying the requesting member and the insurance to a computer of the contractant or insurance company to make a demand for perform a payment processing of the indemnity. The computer of the insurance company received the insurance claim executes the payment processing of the indemnity (Step S179). While the demand of the appropriation to cost or the payment of the indemnity may be performed together after the therapeutic-autologous-cell-based therapy is initiated, the above process capable of performing such operations sequentially and in a short period after receiving the delivery request makes it possible to give the above monetary proof to related organizations in an early stage and to manage the system significantly smoothly.

The delivery source determination means 26 determines a delivery destination from a plurality of pre-registered therapeutic facilities in accordance with the positional information of the requesting member, so as to allow the requesting member to have the therapy adequately, and then determines suitable one of the plurality of depository facilities. The detailed operation of this step will be described as follows. Firstly, among a plurality of pre-registered delivery destinations, the delivery source determination means 26 determines one delivery destination having the highest accessibility to the location of the requesting member in accordance with the positional information of the requesting member. The delivery destination is a place, such as a given hospital, at which the requesting member can receive the therapeutic-autologous-cell-based therapy. Preferably, the information about the determined delivery destination is transmitted and notified to the requesting-member-side such as the member terminal 50 via the communication network 40. This transferred information allows the requesting member with lesions to be reliably transported to the delivery destination such as a hospital to which the therapeutic autologous cell will be delivered. The requesting member may otherwise determine a delivery destination, and transmit the information about the reviver to the delivery-request processing section 20. Then, among the plurality of depository facilities 30 reserving the therapeutic autologous cell of the requesting member, the delivery source determination means 26 determines one depository facility 30 located at the shortest distance to the determined reviver. More preferably, the delivery source determination means 26 determines one depository facility 30 located relative to the delivery destination in a minimum arrival time or in a minimum delivery time. Preferably, in calculation of the arrival time is calculated, an optimal delivery means 62 is selected, and then the traffic condition in route to be used by the delivery means 62 is considered.

The delivery-request transfer means 27 transmits a delivery instruction including information about the requesting member and information about the delivery destination to the transferred-delivery-request receiving means 31 related to the depository facility 30 or delivery source (Step S 183). The delivery instruction received by the transferred-delivery-request receiving means 31 is output to a responsible person through the delivery-request output means 33 (Step S185). The responsible person checks the delivery instruction to prepare the therapeutic autologous cell of the requesting member concerning the delivery instruction from the reserve means 34.

The delivery-request transfer means 27 transmits data on identification-physical characteristics of the requesting member concerning the delivery instruction to the transferred-delivery-request receiving means 31 related to the depository facility 30 or deriver. The identification-physical characteristics data received by the transferred-delivery-request receiving means 31 is output to a responsive person through the member-identification-information output means 32 (Step S 189). The output identification-physical characteristics data represents physical characteristics, such as a facial portrait image, a fingerprint pattern, a vein pattern or an iris pattern, which allow third part to objectively identify the requesting member. The therapeutic autologous cell is delivered to the delivery destination together with the data or information to reliably identify the requesting member at the location of the delivery destination even if the requesting member looses consciousness (Step S 189)

The delivery instruction means 28 determines delivery means 62 for delivering the therapeutic autologous cell in accordance with the positional information of the requesting member, and transmits the delivery instruction to the delivery-instruction receiving means 61 associated with the delivery means 62 (Step S191). Specifically, the delivery instruction means 28 pre-stored all types and locations of delivery means 62 (automobile, two-wheeled vehicle, helicopter etc.), and determines one delivery means 62 close to the requesting member, in accordance with the positional information of the requesting member, to use it in actual delivery. More precisely, in accordance with the information about the delivery source and the delivery destination, the delivery means 62 is preferably selected such that a time-period from the base (delivery handling section 60) of the delivery means 62 to the delivery destination through the delivery source is minimized. Then, the delivery instruction means 28 transmits the delivery instruction including the information about the delivery source and the delivery destination, to the delivery-instruction receiving means 61 associated with the delivery means 62 determined as above. When the delivery instruction is received by the delivery-instruction receiving means 61, a driver of the derivery means 62 can check the content of the delivery instruction, and delivers the therapeutic autologous cell of the requesting member concerning the derivery instruction, from the depository means 34 or delivery source to the delivery destination while driving the derivery means 62 (Step P193). While the derivery means 62 derivers the therapeutic autologous cell while maintaining it under frozen storage, the therapeutic autologous cell is preferably thawed during derivery to provide its adequate state usable for the therapy by an estimated arrival time.

In the therapeutic facility received the therapeutic autologous cell, the therapeutic autologous cell is thawed in the state allowing in-vivo administration, and intravenously administered into the body of the requested member. As mentioned above, the effect is enhanced as the time between the occurrence of lesions and the administration is reduced. The support system 100 according to the present invention can support an adequate and prompt delivery, and allows the therapeutic autologous cell to be quickly delivered to members. Thus, the therapeutic effect on a member will be drastically enhanced.

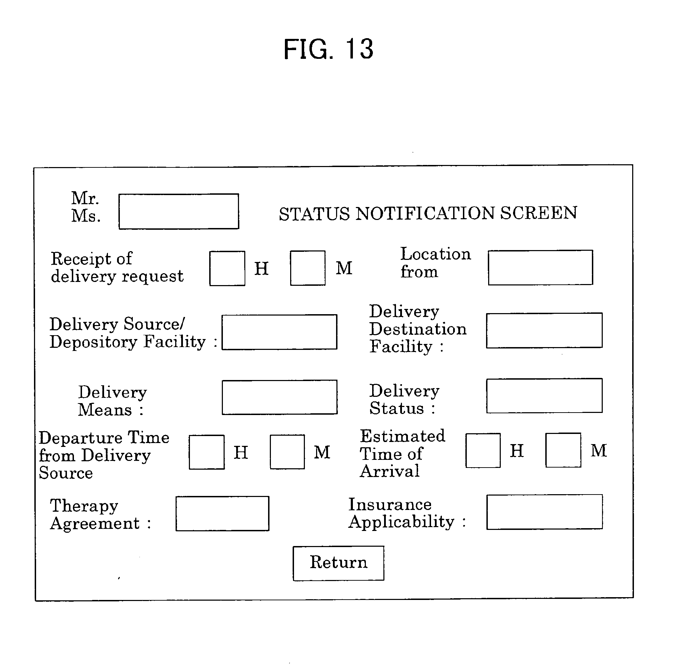

The communication/control means 29 transmits the status of the delivery-request processing section 20 to the member terminal 50 via the communication network 40 (Step S 195). Specifically, the communication/control means 29 transmits information such as the receipt time of a delivery request; the position from which the delivery request is transmitted (position of a member); a depository facility determined as a delivery source; a facility of as the delivery source; derivery means determined by the derivery instruction means 28; status of the derivery (in preparation; ready; delivery (fine); delivery (traffic); done); departure time at delivery source; estimated arrival time to delivery destination (The estimated arrival time is determined from type of deriver means 62, distance, traffic in route etc. Further, the estimated arrival time can be corrected based on information from delivery means 62, in real time); presence of therapy agreement; presence of benefits of insurance (responsible or not), to the member terminal. FIG 13 is an image diagram of a status-notification screen. The steps of this status notification process are operated in parallel with the aforementioned steps, and thereby the participant of the requesting member can figure out the operational status of the delivery-request processing section 20 or the operational progress of delivering the therapeutic autologous cell, in real time.

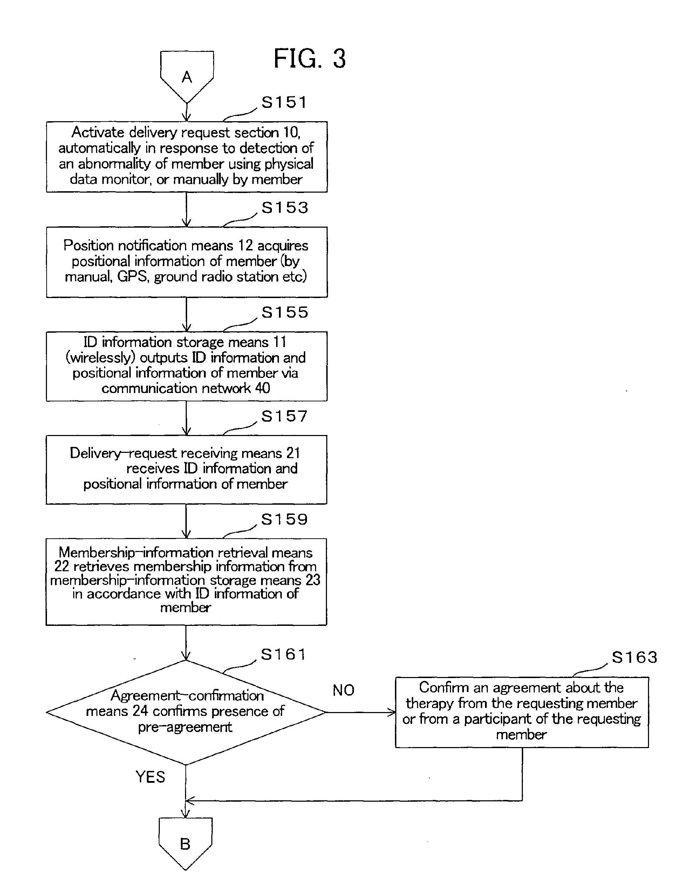

With reference to FIG 21, an operation of the therapeutic autologous-cell delivery support system 101 according to another embodiment of the present invention (hereinafter referred to as "support system 101" for brevity) will be described below. FIG 21 is an exemplary flowchart of a continuous position notification process in the therapeutic autologous-cell delivery support system 101, which substitutes a part of the flowchart in FIG 3. Except that the operation of Steps S201 to S209 illustrated in FIG 21 is substituted for the operation of Steps S151 to S 155 in the support system 100, the operation of the support system 101 is substantially the same as that of the support system 100. Thus, only the different operation will be described below.

A member regularly carries the continuous position notification means 71. The continuous position notification means 71 continuously notifies ID information and positional information of the member to the continuous location receiving means 72 through the communication network 40 (Step S201). The location comparison means 73 compares the positional information of the member with each positional information of facilities, such as medical centers, stored on the facility location storage means 74, to determine whether there is a facility corresponding to the positional information of the member, or whether the member is in one of the facilities having positional information stored in the facility location storage means 74 (Step S203). If there is no corresponding facility, the process will return to Step S201. When there is a corresponding facility, an emergency situation is likely to occur in the member. Thus, the process advances to the next step to confirm whether a delivery request is necessary. In the next step, the location comparison means 73 determines the necessity of delivery in accordance with the secondary index (Step S205). The secondary index may include an expertise index representing degrees of expertise for each of the facilities, and/or a current time. The degree of the necessity of delivery may be increased as the expertise index of the facility has a higher value. If it is late at night or early-morning, the degree of the necessity of delivery may be further increased. If it is determined that the delivery is unnecessary, the process will return to Step S201. When it is determined that the delivery is necessary, the process advances to the next step to determine whether the delivery may be actually transmitted. In the next step, the delivery-request confirmation means determines whether the delivery request may be transmitted (Step S207). The confirmation on transmission of the delivery request may be sought to the member, a responsible person in the facility, an operator in an administrative entity of this system or the like. When the confirmation is sought to the member, the confirmation may be transmitted through telephone or e-mail in accordance with a telephone number or e-mail address pre-registered as the membership information. If it is required to obtain the confirmation from a responsible person in the facility, a telephone number or e-mail address of each facility may be pre-stored on the facility location storage means 74, and the confirmation may be transmitted through telephone or e-mail in accordance with the telephone number or e-mail address read from the facility location storage means 74. In this way, even in the situation where the member cannot make a response, required information can be reliably obtained to adequately determine whether the delivery request may be transmitted. If it is required to obtain the confirmation from an operator in the administrative entity of this system, the delivery-request confirmation means 75 may output the positional information of the member and obtain the confirmation of the delivery request in accordance with a delivery request determined and entered by the operator. Further, the operator may contact with the responsible person in the facility to make the confirmation. If it is confirmed that the delivery request should not be transmitted, the process will return to Step S201. When it is confirmed that the delivery request may be transmitted, the process advances to the next step.

In the next step, the delivery-request confirmation means 75 transmits the delivery request to the delivery-request processing section 20 through the communication network 40 (Step S209). This delivery request may include information about the facility determined that the member has entered therein, such as the name, telephone number and/or vicinity map of the facility. The delivery request may further include similar information about a delivery destination to allow the delivery means 62 to smoothly deliver the therapeutic autologous cells. In this manner, the delivery request of the therapeutic autologous cells for the member will be transmitted. Then, the same steps as Steps S 157 and the subsequent steps will be performed.

According to this embodiment, even if a delivery request is personally transmitted from a member, the necessity of the delivery of therapeutic autologous cells for the member can be autonomously determined based on monitoring of a location of the member.

Positional information of a member to be continuously notified is not essential to be a type directly represented by positional parameters, but may be location-specifying information capable of indirectly representing a location of the member. The location-specifying information can be, for example, a receiving device specifying information as to which of the receiving device receives a signal from the continuous position notification means 71 of the member where a plurality of receiving devices may be set up, respectively, at the facilities. In this case, each location of the facilities having the receiving devices is known in advance. Thus, the receiving device specifying information and the positional information of the corresponding facilities may be pre-stored to determine which of the facilities having the receiving device received a signal from the continuous position notification means 71 of the member.

In the therapeutic autologous-cell delivery support system of the present invention, a therapeutic autologous cell extracted from a member is reserved in reserve means, and an autologous-cell delivery request from the member which includes ID information and positional information is received. Membership information is retrieved in accordance with the received ID information, and the membership information and positional information of the member is output to deliver the autologous cell from a depository facility or delivery source to another facility or delivery destination. Thus, the support system of the present invention can effectively support a regeneration therapy using the reserved autologous cell capable of bringing out a therapeutic effect on emergence lesions with prompt in-vivo administration.

In support system of the present invention, the reserve means may be provided in plural number at geographically different locations, and one reserve means having a shortest delivery time is selected in accordance with the positional information included in the derivery request. Thus, the autologous cell can be advantageously delivered from the nearest one of the reserve means which are dispersedly located around the country. The support system of the present invention may also include ID information storage means for transmitting the ID information manually. Thus, the delivery request can be issued through a simple operation. The delivery request may also be automatically issued using a monitor of physical data. Thus, even if a member is in an emergency condition, or the level of consciousness or physical/ esthesis ability is lowered, the delivery request can be reliably issued. The support system of the present invention may be configured such that when abnormality is detected in the physical data, the information is once output, and the ID information may be transmitted to the communication network only if a confirmation signal is not entered within a given time from the output. Thus, even if the situation where a patient becomes unconsciousness, the delivery request can be automatically issued while preventing any wrong operation. In the support system of the present invention, the position of a member may be automatically acquired through GPS or the like, and transmitted as a part of the delivery request. Thus, the position of the member can be accurately acquired, and the delivery source and delivery destination can be reliably determined. In the support system of the present invention, identification-physical-characteristic data of a member may be stored, and provided to the delivery destination as one of the information of the member. Thus, even if the member looses consciousness, any third party can reliably identify the member. The support system of the present invention may be configured to obtain a pre-agreement. Thus, even if a member looses consciousness in an emergence lesion, the therapy can be initiated without obtaining getting permission of the therapy again. In the support system of the present invention, one suitable delivery means may be determined from a plurality of delivery means located at geographically different positions, and the delivery request is transmitted to the determined delivery means. Thus, the delivery operation can be initiated by selecting delivery means located in the shortest derivery time between the delivery source and the delivery destination. In the support system of the present invention, information, such as the status of derivery process, such as the receipt time of a delivery request; positional information included in the delivery request; reserve means determined as a delivery source; determined delivery destination; determined delivery means; the status of the delivery; the departure time from the delivery source; the estimated arrival time to the delivery destination; the presence of the therapy agreement; and the presence of effective insurance guarantee may be transmitted via the communication network. Thus, a member or participant thereof can confirm the status of the derivery in real time. In the support system of the present invention, various operations, such as, confirmation of intention to become membership; acquirement of membership information; input of questionnaire; and feedback of the questionnaire, may be performed through the communication network. Thus, a series of procedures, such as application for membership; membership registration; inquiry in application; and advice based on the inquiry, can be performed via the Internet or the like. The support system of the present invention may include a dedicated link with the communication network, and may be configured such that the incoming of the delivery request is judged based on the receipt of data. Thus, the system can be reliably activated. The support system of the present invention may include a link with the communication network, and may be configured such that the incoming of the delivery request is judged based on the detection of given data characterizing the delivery request, from received data. Thus, the system can be reliably activated. The support system of the present invention may deliver the therapeutic autologous cell in a short time to effectively perform a therapy of cerebral infarction; cerebral apoplexy including subarachnoid hemorrhage; central and peripheral demyelinating diseases; central and peripheral degenerative diseases; cerebral tumor; disorders of higher brain function including dementia; psychiatric diseases; epilepsy; traumatic neurological diseases including head injury, cerebral contusion and spinal cord injury; inflammatory diseases; and cerebral injury-infectious diseases including Creutzfeldt-Jakob disease. In the support system of the present invention, the therapeutic autologous cell may be prepared by extracting a mesenchymal stem cell from the member in an undifferentiated form, and then genetically modifying the extracted mesenchymal stem cell, or may be prepared by differentiating a mesenchymal stem cell extracted from the member in an undifferentiated form, and inducing the differentiated cell into certain cell. Thus, a regeneration therapy can be used in a wide range.

The financial system of the present invention is configured to make a demand for periodically appropriating a collected fund from a membership fee to an administrative/maintenance cost to be paid on a regular basis, and to make a demand for appropriating the collected fund to a temporary cost including a delivery cost arising from the delivery request. Thus, the collected fund can be appropriated to a regular cost and an incidental cost to allow the system to be managed under monetary proof. The financial system of the present invention may be adapted to make a demand for appropriating the collected fund to a therapeutic cost related to the delivery request. Thus, the collected fund can be appropriated to not only system management cost but also therapeutic cost. The financial system of the present invention may be adapted to make a demand for appropriating an indemnity of an insurance to a therapeutic cost related to the delivery request, wherein at least a part of the collected fund is appropriated to the insurance fee of the insurance. This makes it possible to clearly show how much individual pre-payment is necessary to take an advantage of the inter-complementary indemnification against an incidental cost of an intended therapy, to applicants for membership, in the form of the relationship between an indemnity and an insurance fee. The financial system of the present invention may be adapted to confirm whether effective indemnification is guaranteed by the insurance, in advance, and to give a notification to the requesting member or the participant thereof to understand that no indemnification is guaranteed by the insurance via the communication network. Thus, an opportunity to prepare a fund can be given in an early stage. The financial system of the present invention may be adapted to confirm the intention of each of the members to take out the insurance, and acquire information about an applicant for insurance, via the communication network. Thus, a series of procedure for taking out an insurance can be performed via the Internet or the like. The financial system of the present invention may be adapted to transmit typical insurance designs showing a payment including an insurance fee, a cost to be paid in connection with the delivery request and an indemnity in both cases of taking out the insurance and taking out no insurance, via the communication network. This makes it possible to provide an opportunity to confirm the content of an insurance, and to impress an advantage of the insurance in issuing a delivery request.

Further, according to the present invention, a delivery request including ID information of a member who requires the delivery of the therapeutic autologous cells for the therapeutic purpose and location-specifying information for specifying a location of the member is transmitted via a communication network. This provides an advantage of being able to deliver the therapeutic autologous cells to the member without directly transmitting information about a location of the member, so as to effectively support regenerative therapies using autologous cells. According to the present invention, a location of a member can be determined based on the location-specifying information. This provides an advantage of being able to reliably specify a location of the member. According to the present invention, ID information of a member is stored on an external memory, such as USB key, and preferably a program or transmitting the ID information of the member via a communication network when the external memory device is connected to a computer terminal, or a program for transmitting via a communication network location-specifying information of a computer terminal to which the external memory device is connected. This provides an advantage of being able to utilize a device having excellent portability for the member for the purpose of identifying the member and specifying a location of the member. According to the present invention, the external memory device is configured as a USB key. This provides an advantage of being able to utilize the USB key having excellent versatility and portability for the purpose of identifying the member and specifying a location of the member. According to the present invention, positional information of a member is continuously monitored. This provides an advantage of being able to autonomously determine whether the delivery of therapeutic autologous cells is necessary for the member. According to the present invention, the necessity of a delivery request is determined in accordance with a confirmation on necessity of the delivery request, and a secondary index, such as expertise of a facility and/or current time, in addition to the continuously monitored positional information. This provides an advantage of being able to achieve enhanced accuracy of the determination on necessity of the delivery request.

## Claims

1. A therapeutic autologous-cell delivery support system comprising:
reserve means for reserving a plurality of therapeutic autologous cells which are extracted from the respective bodies of members and then individually propagated up to at least a population required for a therapy of regenerating an in-vivo injury region with in-vivo administration to each of said members, said therapeutic autologous cells being reserved in a distinct manner with respect to each of said members;
computer-based membership-information storage means for storing membership information including at least member's names in such a manner that said member's names are associated, respectively, with ID information uniquely corresponding to said members having said therapeutic autologous cells reserved in said reserve means;
computer-based delivery-request receiving means for receiving a delivery request including the ID information of the member making a request for delivering the therapeutic autologous cell to a delivery destination for the purpose of said therapy, and positional information of said member, via a communication network;
computer-based membership-information retrieval means for retrieving the membership information of said requesting member from said membership-information storage means, in accordance with said ID information included in said delivery request received by said delivery-request receiving means; and
delivery-request output means for outputting information in association with said depositary means, said delivery-request output means being adapted to output said membership information retrieved by said membership-information retrieval means, and said positional information of said requesting member.

2. The therapeutic autologous-cell delivery support system as defined in claim 1, wherein each of said reserve means and said delivery-request output means is provided in a plural number at geographically different locations, wherein said therapeutic autologous-cell delivery support system further includes:
computer-based delivery source determination means for determining the delivery destination from a plurality of pre-registered therapeutic facilities in accordance with the positional information of said requesting member included in said delivery request received by said delivery-request receiving means, so as to allow said requesting member to have the therapy adequately, and then determining suitable one of said plurality of reserve means as the delivery source; and
computer-based delivery-request transfer means for transferring the retrieved membership information from said membership-information retrieval means in the form of including at least the member's name, and delivery destination information of said determined delivery destination, to the delivery-request output means associated with said determined reserve means as the delivery source,
wherein said delivery-request output means is adapted to output said membership information and said delivery destination information transferred from delivery-request transfer means.

3. The therapeutic autologous-cell delivery support system as defined in claim 2, wherein said delivery source determination means is adapted to determine as the delivery source the reserve means which is located at the geographically location allowing a delivery time to said determined delivery destination to be minimized.

4. The therapeutic autologous-cell delivery support system as defined in either one of claims 1 to 3, which further includes ID information storage means for storing ID information of each of said members, and transmitting said ID information to said delivery-request receiving means via said communication network.

5. The therapeutic autologous-cell delivery support system as defined in claim 4, wherein said ID information storage means is adapted to transmit said ID information to said communication network in response to a manual operation of each of said members.

6. The therapeutic autologous-cell delivery support system as defined in claim 4, wherein said ID information storage means is adapted to monitor at least either one of physical data selected from the group consisting of hart rate, breathing rate, electrocardiographic pattern, voice and physical movement, and to transmit said ID information to said communication network in response to the detection of abnormality in said physical data.

7. The therapeutic autologous-cell delivery support system as defined in claim 6, wherein said ID information storage means is adapted to output information about abnormality in said physical data in response to the detection thereof, and subsequently transmit said ID information to said communication network only if no confirmation signal is entered within a predetermined time-period after said output.

8. The therapeutic autologous-cell delivery support system as defined in either one of claims 1 to 7, wherein said ID information storage means is adapted to be wirelessly connected to said communication network.

9. The therapeutic autologous-cell delivery support system as defined in either one of claims 1 to 8, which further includes position notification means for determination the location of said requesting member, and transmit said determined location as the positional information of said requesting member to said delivery-request receiving means via said communication network.

10. The therapeutic autologous-cell delivery support system as defined in claim 9, wherein said position notification means is adapted to be wirelessly connected to said communication network.

11. The therapeutic autologous-cell delivery support system as defined in claim 10, wherein said position notification means is adapted to determine the location of said requesting member according to GPS.

12. The therapeutic autologous-cell delivery support system as defined in claim 10, wherein said position notification means is adapted to receive radiowaves from a ground radio communication facility so as to determine the location of said requesting member.

13. The therapeutic autologous-cell delivery support system as defined in either one of claims 1 to 12, wherein said membership information stored in said membership-information storage means includes at least either one of identification physical-characteristic data selected from the group consisting of a facial portrait image, a fingerprint pattern, a vein pattern and an iris pattern.

14. The therapeutic autologous-cell delivery support system as defined in either one of claims 1 to 13, which further includes computer-based agreement-confirmation means for confirming the presence of a pre-agreement of each of said members to use the therapeutic autologous cell, via said communication network, wherein said delivery-request output means is adapted to output the membership information and the positional information of said requesting member if the pre-agreement is confirmed to have been obtained from said requesting member, and to output the membership information and the positional information of said requesting member only after the agreement is obtained from said requesting member or a given participant of said requesting member if the pre-agreement is not confirmed to have been obtained form said requesting member.

15. The therapeutic autologous-cell delivery support system as defined in either one of claims 1 to 14, which further includes delivery instruction means for determining one of a plurality of delivery means provided at geographically different locations to deriver the therapeutic autologous cell, in accordance with the positional information of said requesting member, and transmitting a delivery instruction to said determined delivery means.

16. The therapeutic autologous-cell delivery support system as defined in claim 15, which further includes status notification means for transmitting at least either one of information selected from the group consisting of the receipt time of the delivery request, the positional information included in the delivery request, the determined reserve means as the delivery source, the determined delivery means, the status of the delivery, the departure time from the delivery source, the estimated arrival time to the delivery destination, the presence of the therapy agreement and the presence of effective insurance guarantee, to said requesting member or a given participant of said requesting member via said communication network.

17. The therapeutic autologous-cell delivery support system as defined in either one of claims 1 to 16, which further includes computer-based membership-application acceptance means for confirming the intention of an applicant to become a member, via said communication network, acquiring information including at least the name of said applicant as said membership information, via said communication network, allowing said applicant to enter a response to a questionnaire thereto via said communication network, and providing a feedback based on said questionnaire response to said applicant via said communication network.

18. The therapeutic autologous-cell delivery support system as defined in either one of claims 1 to 17, wherein said delivery-request receiving means includes a dedicated link with said communication network to receive the delivery request, so as to allow the incoming of said delivery request to be judged in accordance with the fact that any data is received through said dedicated link.

19. The therapeutic autologous-cell delivery support system as defined in either one of claims 1 to 17, wherein said delivery-request receiving means includes a link with said communication network to detect given data characterizing the delivery request, so as to allow the incoming of said delivery request to be judged in accordance with the detection of said given data.

20. The therapeutic autologous-cell delivery support system as defined in either one of claims 1 to 19, wherein said therapeutic autologous cell is used in at least either one of therapies selected from the group consisting of: cerebral infarction; cerebral hemorrhage; vertebral injury; cardiac infarction; cerebral apoplexy including subarachnoid hemorrhage; central and peripheral demyelinating diseases; central and peripheral degenerative diseases; cerebral tumor; disorders of higher brain function including dementia; psychiatric diseases; epilepsy; traumatic neurological diseases including head injury, cerebral contusion and spinal cord injury; inflammatory diseases; and cerebral injury-infectious diseases including Creutzfeldt-Jakob disease.

21. The therapeutic autologous-cell delivery support system as defined in either one of claims 1 to 20, wherein said therapeutic autologous cell is a mesenchymal stem cell extracted from the member in an undifferentiated form.

22. The therapeutic autologous-cell delivery support system as defined in either one of claims 1 to 20, wherein said therapeutic autologous cell is prepared by extracting a mesenchymal stem cell from the member in an undifferentiated form, and then genetically modifying said extracted mesenchymal stem cell.

23. The therapeutic autologous-cell delivery support system as defined in either one of claims 1 to 20, wherein said therapeutic autologous cell is prepared by differentiating a mesenchymal stem cell extracted from the member in an undifferentiated form, and inducing the differentiated cell into a certain cell.

24. A financial system for use with the therapeutic autologous-cell delivery support system as defined in either one of claims 1 to 21, said financial system comprising:
computer-based cost-appropriation demand means for making a demand for appropriating a collected found from the member to an owing cost, said cost-appropriation demand means being adapted to periodically make a demand for appropriating said collected fund to an administrative/maintenance cost to be paid on a regular basis, and making a demand for appropriating said collected fund to a temporary cost including a delivery cost arising from the delivery request.

25. The financial system as defined in claim 24, wherein said cost-appropriation demand means is adapted to make a demand for appropriating said collected fund to a therapeutic cost related to the delivery request.

26. The financial system as defined in claim 24, wherein said cost-appropriation demand means is adapted to make a demand for appropriating an indemnity of an insurance to a therapeutic cost related to the delivery request, wherein at least a part of said collected fund is appropriated to the insurance fee of said insurance.

27. The financial system as defined in claim 26, wherein said cost-appropriation demand means is adapted to confirm whether effective indemnification is guaranteed by said insurance, in advance in accordance with the provisions of said insurance and the reason of said delivery request, when demanding for appropriating the insurance indemnity to the therapeutic cost related to said delivery request, and to give a notification to said requesting member or a participant thereof to understand that no indemnification is guaranteed by said insurance via said communication network.

28. The financial system as defined in claim 26 or 27, which further includes computer-based insurance-application acceptance means for confirming the intention of taking out the insurance, and acquiring at least a part of given information necessary for taking out said insurance, via said communication network.

29. The financial system as defined in either one of claims 26 to 28, which further includes insurance-design providing means for transmitting typical insurance designs showing a payment including an insurance fee, a cost to be paid in connection with the delivery request and an indemnity in both cases of taking out the insurance and taking out no insurance, via said communication network.

30. A method for supporting the delivery of therapeutic autologous cells, comprising the steps of:
reserving a plurality of therapeutic autologous cells which are extracted from the respective bodies of members and then individually propagated up to at least a population required for a therapy of regenerating an in-vivo injury region with in-vivo administration to each of said members, in reserve means in a distinct manner with respect to each of said members;
storing membership information including at least member's names in such a manner that said member's names are associated, respectively, with ID information uniquely corresponding to said members having said therapeutic autologous cells reserved in said reserve means, according to a computer;
receiving a delivery request including the ID information of the member making a request for delivering the therapeutic autologous cell to a delivery destination for the purpose of said therapy, and positional information of said member, via a communication network according to a computer;
retrieving the membership information of said requesting member from said membership-information storage means, in accordance with said ID information included in said delivery request received in said delivery-request receiving step, according to a computer; and
outputting information in association with said depositary means, said information including said membership information retrieved in said membership-information retrieving step, and said positional information of said requesting member.

31. A financing method for used with the method as defined in claim 30, comprising the step of making a demand for appropriating a collected fund from the member to an owing cost, according to a computer, wherein a demand for appropriating said collected fund to an administrative/maintenance cost to be paid on a regular basis is periodically made, and a demand for appropriating said collected fund to a temporary cost including a delivery cost arising from the delivery request.

32. The financing method as defined in claim 31, which includes making a demand for appropriating said collected fund to a therapeutic cost related to the delivery request.

33. The financing method as defined in claim 32, which includes making a demand for appropriating an indemnity of an insurance to a therapeutic cost related to the delivery request,
wherein at least a part of said collected fund is appropriated to the insurance fee of said insurance.

34. A therapeutic autologous-cell delivery support system comprising:
reserve means reserving therapeutic autologous cells which are extracted from each body of a plurality of members and then propagated up to at least a population required for a therapy to regenerate an in-vivo injury region with in-vivo administration to each of the members, distinctively on a member-by-member basis;
computer-based membership-information storage means storing a plurality of ID information uniquely corresponding to said respective members having said therapeutic autologous cells reserved in said reserve means, and each membership information of said members which includes at least each name of said members, in a mutually associated manner;
computer-based delivery-request receiving means for receiving via a communication network a delivery request which includes the ID information of a specific one of said members who requires the delivery of said therapeutic autologous cell to a delivery destination for said therapeutic purpose, and location-specifying information for specifying a location of said requesting member;
computer-based membership-information retrieval means for retrieving the membership information of said requesting member from said membership-information storage means, in accordance with said ID information included in said delivery request received by said delivery-request receiving means; and
delivery-request output means association with said depositary means and adapted to output said membership information retrieved by said membership-information retrieval means, and said location-specifying information of said requesting member.

35. The therapeutic autologous-cell delivery support system as defined in claim 34, which further includes location determination means for determining a location of said requesting member in accordance with said location-specifying information.

36. The therapeutic autologous-cell delivery support system as defined in claim 35, wherein each of said reserve means and said delivery-request output means is provided in a plural number and situated at geographically different locations, wherein said therapeutic autologous-cell delivery support system further includes:
computer-based delivery source determination means for specifying one of a plurality of pre-registered facilities including a therapeutic facility, as a suitable delivery destination for said requesting member to receive the therapy, in accordance with the location of said requesting member determined by said location determination means, and then selecting one of said plurality of reserve means, as a suitable delivery source; and
computer-based delivery-request transfer means for transferring the membership information with at least member's name, retrieved by said membership-information retrieval means, and delivery destination information about said specified delivery destination, to one of said plurality of delivery-request output means which is associated with said selected reserve means as the suitable delivery source, said associated delivery-request output means being operable to output said membership information and said delivery destination information transferred from said delivery-request transfer means.

37. The therapeutic autologous-cell delivery support system as defined in claim 36, wherein said reserve means selected as the suitable delivery source by said delivery source determination means is situated at a topographical location allowing a delivery time to said specified delivery destination to be minimized.

38. The therapeutic autologous-cell delivery support system as defined in either one of claims 34 to 37, which further includes ID information storage means storing the ID information of said specific member, said ID information storage means being adapted to transmit said ID information to said delivery-request receiving means via said communication network.

39. The therapeutic autologous-cell delivery support system as defined in claim 38, wherein said ID information storage means comprises an external memory device storing said ID information thereon.

40. The therapeutic autologous-cell delivery support system as defined in claim 39, wherein said external memory device further stores an ID information transmission program to be executed in response to connecting said ID information storage means to a computer terminal, so as to transmit said member's ID information to said delivery-request receiving means via said communication network.

41. The therapeutic autologous-cell delivery support system as defined in claim 39, wherein said external memory device further stores a location-specifying information transmission program to be executed in response to connecting said ID information storage means to a computer terminal, so as to transmit a location of said computer terminal as said location-specifying information for specifying each location of the members, to said delivery-request receiving means via said communication network.

42. The therapeutic autologous-cell delivery support system as defined in claim 41, wherein said location-specifying information transmission program is operable, when executed on the computer terminal, to prompt an operator of said computer terminal to enter information about a location of said operator, and then transmit said entered information as said location-specifying information to said delivery-request receiving means.

43. The therapeutic autologous-cell delivery support system as defined in either one of claims 39 to 42, wherein said external memory device is configured as a USB key.

44. A therapeutic autologous-cell delivery support system comprising:
reserve means reserving therapeutic autologous cells which are extracted from each body of a plurality of members and then propagated up to at least a population required for a therapy to regenerate an in-vivo injury region with in-vivo administration to each of the members, distinctively on a member-by-member basis;
computer-based membership-information storage means storing a plurality of ID information uniquely corresponding to said respective members having said therapeutic autologous cells reserved in said reserve means, and each membership information of said members which includes at least each name of said members, in a mutually associated manner;
computer-based continuously-operative location receiving means for continuously receiving location-specifying information for specifying each location of said members, via a communication network;
facility-location storage means storing information about a plurality of facilities and each location of said facilities, in a mutually associated manner;
location comparison means for comparing the location specified by said location-specifying information received from a specific one of said members with each location of said facilities stored on said facility-location storage means to determine whether there is a facility corresponding to the location of said specific member;
delivery-request transmission means operable, in response to the determination of said location comparison means that there is a facility corresponding to the location of said specific member, to transmit a delivery request including the ID information and the location-specifying information of said specific member;
computer-based membership-information retrieval means for retrieving the membership information of said specific member from said membership-information storage means, in accordance with said ID information transmitted from said delivery-request transmission means; and
delivery-request output means association with said depositary means and adapted to output said membership information retrieved by said membership-information retrieval means, and said location-specifying information of said specific member.

45. The therapeutic autologous-cell delivery support system as defined in claim 44, wherein said delivery-request transmission means is adapted to, before transmission of the delivery request, confirm whether the delivery request for said specific member may be transmitted.

46. The therapeutic autologous-cell delivery support system as defined in claim 44, wherein said delivery-request transmission means is adapted to, before transmission of the delivery request, determine whether the delivery request should be transmitted, in accordance with a secondary index including at least either one of each expertise index of said facilities and a current time.
